# EUROPEAN PATENT APPLICATION

(11) **EP 3 583 949 A1**
(43) Date of publication of application: **25.12.2019**
(21) Application number: 18178651.8
(22) Date of filing: 19.06.2018
(51) Int. Cl.: A61K 39/395, A61K 45/06, A61P 35/04, A61P 35/00

(54) **TOLL-LIKE RECEPTOR 2 (TLR2) ANTAGONISTS FOR THE TREATMENT OF OVARIAN CANCER**

(71) Applicant: Opsona Therapeutics Limited, Dublin 2 (IE)
(72) Inventor: KEOGH, Brian, Dublin 2 (IE)
(74) Representative: Murgitroyd & Company

(57) **Abstract**

A composition comprising a TLR2 antagonistic antibody or antigen binding fragment thereof for use in the treatment or prophylaxis of ovarian cancer is provided. Also provided is a screening method for the identification of compounds for use in treatment or prevention of ovarian cancer.

SEQUENCE LISTING

<110>  Opsona Therapeutics Limited

<120>  Methods and Compositions for the Treatment of Ovarian Cancer

<130>  P194963.EP.01

<150>
<151>

<160>  13

<170>  PatentIn version 3.5

<210>  1
<211>  784
<212>  PRT
<213>  Homo sapiens

<400>  1

<210>  2
<211>  784
<212>  PRT
<213>  Mus musculus

<400>  2

Met Leu Arg Ala Leu Trp Leu Phe Trp Ile Leu Val Ala Ile Thr Val
1               5                   10                  15

Leu Phe Ser Lys Arg Cys Ser Ala Gln Glu Ser Leu Ser Cys Asp Ala
            20                  25                  30

Ser Gly Val Cys Asp Gly Arg Ser Arg Ser Phe Thr Ser Ile Pro Ser
        35                  40                  45

Gly Leu Thr Ala Ala Met Lys Ser Leu Asp Leu Ser Phe Asn Lys Ile
    50                  55                  60

Thr Tyr Ile Gly His Gly Asp Leu Arg Ala Cys Ala Asn Leu Gln Val
65                  70                  75                  80

Leu Met Leu Lys Ser Ser Arg Ile Asn Thr Ile Glu Gly Asp Ala Phe
                85                  90                  95

Tyr Ser Leu Gly Ser Leu Glu His Leu Asp Leu Ser Asp Asn His Leu
            100                 105                 110

Ser Ser Leu Ser Ser Ser Trp Phe Gly Pro Leu Ser Ser Leu Lys Tyr
        115                 120                 125

Leu Asn Leu Met Gly Asn Pro Tyr Gln Thr Leu Gly Val Thr Ser Leu
    130                 135                 140

Phe Pro Asn Leu Thr Asn Leu Gln Thr Leu Arg Ile Gly Asn Val Glu
145                 150                 155                 160

Thr Phe Ser Glu Ile Arg Arg Ile Asp Phe Ala Gly Leu Thr Ser Leu
                165                 170                 175

Asn Glu Leu Glu Ile Lys Ala Leu Ser Leu Arg Asn Tyr Gln Ser Gln
            180                 185                 190

Ser Leu Lys Ser Ile Arg Asp Ile His His Leu Thr Leu His Leu Ser
        195                 200                 205

Glu Ser Ala Phe Leu Leu Glu Ile Phe Ala Asp Ile Leu Ser Ser Val
    210                 215                 220

Arg Tyr Leu Glu Leu Arg Asp Thr Asn Leu Ala Arg Phe Gln Phe Ser
225                 230                 235                 240

Pro Leu Pro Val Asp Glu Val Ser Ser Pro Met Lys Lys Leu Ala Phe
                245                 250                 255

Arg Gly Ser Val Leu Thr Asp Glu Ser Phe Asn Glu Leu Leu Lys Leu
            260                 265                 270

Leu Arg Tyr Ile Leu Glu Leu Ser Glu Val Glu Phe Asp Asp Cys Thr
        275                 280                 285

Leu Asn Gly Leu Gly Asp Phe Asn Pro Ser Glu Ser Asp Val Val Ser
    290                 295                 300

Glu Leu Gly Lys Val Glu Thr Val Thr Ile Arg Arg Leu His Ile Pro
305                 310                 315                 320

Gln Phe Tyr Leu Phe Tyr Asp Leu Ser Thr Val Tyr Ser Leu Leu Glu
                325                 330                 335

Lys Val Lys Arg Ile Thr Val Glu Asn Ser Lys Val Phe Leu Val Pro
            340                 345                 350

Cys Ser Phe Ser Gln His Leu Lys Ser Leu Glu Phe Leu Asp Leu Ser
        355                 360                 365

Glu Asn Leu Met Val Glu Glu Tyr Leu Lys Asn Ser Ala Cys Lys Gly
    370                 375                 380

Ala Trp Pro Ser Leu Gln Thr Leu Val Leu Ser Gln Asn His Leu Arg
385                 390                 395                 400

Ser Met Gln Lys Thr Gly Glu Ile Leu Leu Thr Leu Lys Asn Leu Thr
                405                 410                 415

Ser Leu Asp Ile Ser Arg Asn Thr Phe His Pro Met Pro Asp Ser Cys
            420                 425                 430

Gln Trp Pro Glu Lys Met Arg Phe Leu Asn Leu Ser Ser Thr Gly Ile
        435                 440                 445

Arg Val Val Lys Thr Cys Ile Pro Gln Thr Leu Glu Val Leu Asp Val
    450                 455                 460

Ser Asn Asn Asn Leu Asp Ser Phe Ser Leu Phe Leu Pro Arg Leu Gln
465                 470                 475                 480

Glu Leu Tyr Ile Ser Arg Asn Lys Leu Lys Thr Leu Pro Asp Ala Ser
                485                 490                 495

Leu Phe Pro Val Leu Leu Val Met Lys Ile Arg Glu Asn Ala Val Ser
            500                 505                 510

Thr Phe Ser Lys Asp Gln Leu Gly Ser Phe Pro Lys Leu Glu Thr Leu
        515                 520                 525

Glu Ala Gly Asp Asn His Phe Val Cys Ser Cys Glu Leu Leu Ser Phe
    530                 535                 540

Thr Met Glu Thr Pro Ala Leu Ala Gln Ile Leu Val Asp Trp Pro Asp
545                 550                 555                 560

Ser Tyr Leu Cys Asp Ser Pro Pro Arg Leu His Gly His Arg Leu Gln
                565                 570                 575

Asp Ala Arg Pro Ser Val Leu Glu Cys His Gln Ala Ala Leu Val Ser
            580                 585                 590

Gly Val Cys Cys Ala Leu Leu Leu Leu Ile Leu Leu Val Gly Ala Leu
        595                 600                 605

Cys His His Phe His Gly Leu Trp Tyr Leu Arg Met Met Trp Ala Trp

610                 615                 620

Leu Gln Ala Lys Arg Lys Pro Lys Lys Ala Pro Cys Arg Asp Val Cys
625                 630                 635                 640

Tyr Asp Ala Phe Val Ser Tyr Ser Glu Gln Asp Ser His Trp Val Glu
                645                 650                 655

Asn Leu Met Val Gln Gln Leu Glu Asn Ser Asp Pro Pro Phe Lys Leu
            660                 665                 670

Cys Leu His Lys Arg Asp Phe Val Pro Gly Lys Trp Ile Ile Asp Asn
        675                 680                 685

Ile Ile Asp Ser Ile Glu Lys Ser His Lys Thr Val Phe Val Leu Ser
    690                 695                 700

Glu Asn Phe Val Arg Ser Glu Trp Cys Lys Tyr Glu Leu Asp Phe Ser
705                 710                 715                 720

His Phe Arg Leu Phe Asp Glu Asn Asn Asp Ala Ala Ile Leu Val Leu
                725                 730                 735

Leu Glu Pro Ile Glu Arg Lys Ala Ile Pro Gln Arg Phe Cys Lys Leu
            740                 745                 750

Arg Lys Ile Met Asn Thr Lys Thr Tyr Leu Glu Trp Pro Leu Asp Glu
        755                 760                 765

Gly Gln Gln Glu Val Phe Trp Val Asn Leu Arg Thr Ala Ile Lys Ser
    770                 775                 780

<210>  3
<211>  8
<212>  PRT
<213>  Mus musculus

<400>  3

Gly Phe Thr Phe Thr Thr Tyr Gly
1               5

<210>  4
<211>  8
<212>  PRT
<213>  Mus musculus

<400>  4

Ile Tyr Pro Arg Asp Gly Ser Thr
1               5

<210>  5
<211>  11
<212>  PRT
<213>  Mus musculus

<400>  5

Ala Arg Leu Thr Gly Gly Thr Phe Leu Asp Tyr
1               5                   10


<210>  6
<211>  10
<212>  PRT
<213>  Mus musculus

<400>  6

Glu Ser Val Glu Tyr Tyr Gly Thr Ser Leu
1               5                   10


<210>  7
<211>  9
<212>  PRT
<213>  Mus musculus

<400>  7

Gln Gln Ser Arg Lys Leu Pro Trp Thr
1               5


<210>  8
<211>  167
<212>  PRT
<213>  Mus musculus

<400>  8

Met Ser Ser Pro Gln Ser Leu Lys Thr Leu Ile Leu Thr Met Gly Trp
1               5                   10                  15

Ser Trp Ile Phe Leu Phe Leu Leu Ser Gly Thr Ala Gly Val His Ser
            20                  25                  30

Gln Val Gln Leu Gln Gln Ser Gly Pro Glu Leu Val Asn Pro Gly Ala
        35                  40                  45

Ser Val Lys Leu Ser Cys Lys Ala Ser Gly Phe Thr Phe Thr Thr Tyr
    50                  55                  60

Gly Ile Asn Trp Val Lys Gln Gly Pro Gly Gln Gly Leu Glu Trp Ile
65                  70                  75                  80

Gly Trp Ile Tyr Pro Arg Asp Gly Ser Thr Asn Phe Asn Glu Asn Phe

85                  90                  95

Lys Asp Lys Ala Ala Leu Thr Val Asp Thr Ser Ser Ser Thr Ala Tyr
            100                 105                 110

Met Glu Leu His Ser Leu Thr Ser Glu Asp Ser Ala Val Tyr Phe Cys
        115                 120                 125

Ala Arg Leu Thr Gly Gly Thr Phe Leu Asp Tyr Trp Gly Gln Gly Thr
    130                 135                 140

Thr Leu Thr Val Ser Ser Ala Lys Thr Thr Pro Pro Ser Val Tyr Pro
145                 150                 155                 160

Leu Ala Pro Gly Ser Ala Ala
                165

<210>  9
<211>  147
<212>  PRT
<213>  Mus musculus

<400>  9

Met Glu Ser Asp Thr Leu Leu Leu Trp Val Leu Leu Leu Trp Val Pro
1               5                   10                  15

Gly Ser Thr Gly Asp Ile Val Leu Thr Gln Ser Pro Ala Ser Leu Ala
            20                  25                  30

Val Ser Leu Gly Gln Arg Ala Thr Ile Ser Cys Arg Ala Ser Glu Ser
        35                  40                  45

Val Glu Tyr Tyr Gly Thr Ser Leu Met Gln Trp Tyr Gln Gln Lys Pro
    50                  55                  60

Gly Gln Pro Pro Lys Leu Leu Ile Phe Gly Ala Ser Asn Val Glu Ser
65                  70                  75                  80

Gly Val Pro Val Arg Phe Ser Gly Ser Gly Ser Gly Thr Asp Phe Ser
                85                  90                  95

Leu Asn Ile His Pro Val Glu Glu Asp Asp Ile Val Met Tyr Phe Cys
            100                 105                 110

Gln Gln Ser Arg Lys Leu Pro Trp Thr Phe Gly Gly Gly Thr Lys Leu
        115                 120                 125

Glu Ile Lys Arg Ala Asp Ala Ala Pro Thr Val Ser Ile Phe Pro Pro

130                 135                 140

Ser Ser Glu
145

<210>  10
<211>  111
<212>  PRT
<213>  Homo sapiens

<400>  10

Asp Ile Val Leu Thr Gln Ser Pro Ala Thr Leu Ser Leu Ser Pro Gly
1               5                   10                  15

Glu Arg Ala Thr Leu Ser Cys Arg Ala Ser Glu Ser Val Glu Tyr Tyr
            20                  25                  30

Gly Thr Ser Leu Met Gln Trp Tyr Gln Gln Lys Pro Gly Gln Pro Pro
        35                  40                  45

Lys Leu Leu Ile Phe Gly Ala Ser Asn Val Glu Ser Gly Val Pro Asp
    50                  55                  60

Arg Phe Ser Gly Ser Gly Ser Gly Thr Asp Phe Thr Leu Lys Ile Ser
65                  70                  75                  80

Arg Val Glu Ala Glu Asp Val Gly Met Tyr Phe Cys Gln Gln Ser Arg
                85                  90                  95

Lys Leu Pro Trp Thr Phe Gly Gly Gly Thr Lys Val Glu Ile Lys
            100                 105                 110

<210>  11
<211>  118
<212>  PRT
<213>  Homo sapiens

<400>  11

Gln Val Gln Leu Val Gln Ser Gly Ser Glu Leu Lys Lys Pro Gly Ala
1               5                   10                  15

Ser Val Lys Leu Ser Cys Lys Ala Ser Gly Phe Thr Phe Thr Thr Tyr
            20                  25                  30

Gly Ile Asn Trp Val Arg Gln Ala Pro Gly Gln Gly Leu Glu Trp Ile
        35                  40                  45

Gly Trp Ile Tyr Pro Arg Asp Gly Ser Thr Asn Phe Asn Glu Asn Phe
    50                  55                  60

Lys Asp Arg Ala Thr Ile Thr Val Asp Thr Ser Ala Ser Thr Ala Tyr
65                  70                  75                  80

Met Glu Leu Ser Ser Leu Arg Ser Glu Asp Thr Ala Val Tyr Phe Cys
                85                  90                  95

Ala Arg Leu Thr Gly Gly Thr Phe Leu Asp Tyr Trp Gly Gln Gly Thr
            100                 105                 110

Thr Val Thr Val Ser Ser
        115

<210>  12
<211>  218
<212>  PRT
<213>  Homo sapiens

<400>  12

Asp Ile Val Leu Thr Gln Ser Pro Ala Thr Leu Ser Leu Ser Pro Gly
1               5                   10                  15

Glu Arg Ala Thr Leu Ser Cys Arg Ala Ser Glu Ser Val Glu Tyr Tyr
            20                  25                  30

Gly Thr Ser Leu Met Gln Trp Tyr Gln Gln Lys Pro Gly Gln Pro Pro
        35                  40                  45

Lys Leu Leu Ile Phe Gly Ala Ser Asn Val Glu Ser Gly Val Pro Asp
    50                  55                  60

Arg Phe Ser Gly Ser Gly Ser Gly Thr Asp Phe Thr Leu Lys Ile Ser
65                  70                  75                  80

Arg Val Glu Ala Glu Asp Val Gly Met Tyr Phe Cys Gln Gln Ser Arg
                85                  90                  95

Lys Leu Pro Trp Thr Phe Gly Gly Gly Thr Lys Val Glu Ile Lys Arg
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys
    210                 215

<210>  13
<211>  445
<212>  PRT
<213>  Homo sapiens

<400>  13

Gln Val Gln Leu Val Gln Ser Gly Ser Glu Leu Lys Lys Pro Gly Ala
1               5                   10                  15

Ser Val Lys Leu Ser Cys Lys Ala Ser Gly Phe Thr Phe Thr Thr Tyr
            20                  25                  30

Gly Ile Asn Trp Val Arg Gln Ala Pro Gly Gln Gly Leu Glu Trp Ile
        35                  40                  45

Gly Trp Ile Tyr Pro Arg Asp Gly Ser Thr Asn Phe Asn Glu Asn Phe
    50                  55                  60

Lys Asp Arg Ala Thr Ile Thr Val Asp Thr Ser Ala Ser Thr Ala Tyr
65                  70                  75                  80

Met Glu Leu Ser Ser Leu Arg Ser Glu Asp Thr Ala Val Tyr Phe Cys
                85                  90                  95

Ala Arg Leu Thr Gly Gly Thr Phe Leu Asp Tyr Trp Gly Gln Gly Thr
            100                 105                 110

Thr Val Thr Val Ser Ser Ala Ser Thr Lys Gly Pro Ser Val Phe Pro
        115                 120                 125

Leu Ala Pro Cys Ser Arg Ser Thr Ser Glu Ser Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Lys Thr Tyr Thr Cys Asn Val Asp His Lys Pro Ser
        195                 200                 205

Asn Thr Lys Val Asp Lys Arg Val Glu Ser Lys Tyr Gly Pro Pro Cys
    210                 215                 220

Pro Pro Cys Pro Ala Pro Glu Phe Leu Gly Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser Gln Glu Asp Pro Glu Val Gln
            260                 265                 270

Phe Asn Trp Tyr Val Asp Gly Val Glu Val His Asn Ala Lys Thr Lys
        275                 280                 285

Pro Arg Glu Glu Gln Phe Asn Ser Thr Tyr Arg Val Val Ser Val Leu
    290                 295                 300

Thr Val Leu His Gln Asp Trp Leu Asn Gly Lys Glu Tyr Lys Cys Lys
305                 310                 315                 320

Val Ser Asn Lys Gly Leu Pro Ser Ser Ile Glu Lys Thr Ile Ser Lys
                325                 330                 335

Ala Lys Gly Gln Pro Arg Glu Pro Gln Val Tyr Thr Leu Pro Pro Ser
            340                 345                 350

Gln Glu Glu Met Thr Lys Asn Gln Val Ser Leu Thr Cys Leu Val Lys
        355                 360                 365

Gly Phe Tyr Pro Ser Asp Ile Ala Val Glu Trp Glu Ser Asn Gly Gln
    370                 375                 380

Pro Glu Asn Asn Tyr Lys Thr Thr Pro Pro Val Leu Asp Ser Asp Gly
385                 390                 395                 400

Ser Phe Phe Leu Tyr Ser Arg Leu Thr Val Asp Lys Ser Arg Trp Gln

                405                 410                 415

Glu Gly Asn Val Phe Ser Cys Ser Val Met His Glu Ala Leu His Asn
            420                 425                 430

His Tyr Thr Gln Lys Ser Leu Ser Leu Ser Leu Gly Lys
        435                 440                 445

## Description

### Field of the Invention

The present invention relates to methods for the treatment or prevention of ovarian cancer.

### Background to the Invention

Toll-like Receptors (TLRs) form a family of pattern recognition receptors which have a key role in activating the innate immune response. Eleven TLRs have been identified in humans to date. The members of the TLR family are highly conserved, with most mammalian species having between ten to fifteen TLRs. Each TLR recognises specific pathogen-associated molecular signatures. Toll-like Receptor 2 (TLR2, CD282, TLR-2) is activated by peptidoglycan, lipoproteins and lipoteichoic acid. TLR2 is known to dimerise into two functional heterodimers. In particular, TLR2 is known to form a heterodimer with either Toll-like Receptor 1 (TLR1, TLR-1) or Toll-like Receptor 6 (TLR6, TLR-6). It is possible that further heterodimers are formed with Toll-like Receptor 4 (TLR4, TLR-4) and Toll-like Receptor 10 (TLR10, TLR-10). In addition to microbial derived components, TLRs are also known to recognize damage-associated molecular patterns (DAMPs). These are host endogenous molecules released and distributed following stress, tissue damage and cellular disease. WO 2005/028509 discloses a murine IgG1 anti-TLR2 antibody which was derived from hybridoma clone T2.5 (HyCult Biotechnology b.v., Cell Sciences, Canton, USA: catalogue number 1054 - also known as OPN-301). WO2011/003925 describes a humanized version of T2.5 which is designated OPN-305.

Despite an initial 80% response rate, the 5-year survival rate for ovarian cancer is only 15% as most patients develop recurrence. In the recurrent setting, micrometastasis and chemoresistance limits the value of available treatment options (Markman M. Int J Gynecol Cancer. 2009;19 Suppl 2:S40-3; Ushijima K. J Oncol. 2010;2010:497429; Jelovac D, Armstrong DK. CA Cancer J Clin. 2011;61(3):183-203) Therefore, to improve survival it is critical to understand the mechanisms that promote recurrence and to develop novel approaches to prevent it.

There is a need for improved methods for treating ovarian cancer, in particular, recurrent ovarian cancer.

### Summary of the Invention

As detailed in the Examples, the inventors have surprisingly shown using an anti-TLR2 antibody, that by inhibiting TLR2, ovarian cancer tumour size and progression may be inhibited.

Accordingly, in a first aspect of the present invention, there is provided a composition comprising a Toll-like receptor 2 (TLR2) antagonist for use in the treatment of ovarian cancer in a subject.

According to a second aspect of the present invention there is provided a method of treating or preventing ovarian cancer comprising the step of:
administering a therapeutically effective amount of a Toll-like receptor 2 (TLR2) antagonist to a subject in need thereof wherein the TLR2 antagonist is an antibody or an antigen binding fragment thereof.

As detailed below, the inventors have shown that the TLR2 inhibitors were particularly effective in delaying or inhibiting progression of recurrent cancer.

Accordingly, in particular embodiments of the invention, said treatment is treatment or prevention of recurrent ovarian cancer. Such treatment may be provided together with or after surgery and/or treatment with one or more chemotherapeutic agents.

In a particular embodiment, the TLR2 antagonist is for administration as maintenance therapy after cessation of treatment with a chemotherapeutic agent and/or after surgery.

In embodiments involving combination treatment with one or chemotherapeutic agents, any chemotherapeutic agent suitable for the treatment of ovarian cancer may be used. For example, in one such embodiment, the one or more chemotherapeutic agents may be selected from taxanes and platinum containing chemotherapeutic agents. In a particular embodiment, said combination treatment is combination treatment with paclitaxel.

In the invention, any suitable TLR2 antagonist may be used. In certain embodiments. the TLR2 antagonist is an antibody or an antigen binding fragment thereof.

In an embodiment, the antibody or antigen binding fragment thereof specifically binds to an epitope comprising leucine rich repeat regions 11 to 14 of TLR2. Optionally, the antibody or an antigen binding fragment thereof specifically binds to a non-continuous epitope comprising amino acid residues His318, Pro320, Arg321, Tyr323, Lys347, Phe349, Leu371, Glu375, Tyr376 and His398 of SEQ ID NO: 1, or comprising amino acid residues His318, Pro320, Gln321, Tyr323, Lys347, Phe349, Leu371, Glu375, Tyr376 and His398 of SEQ ID NO: 2. In certain embodiments, the antibody or antigen binding fragment comprises a heavy chain variable region comprising a complementarity determining region (CDR) 1 region comprising the amino acid sequence of SEQ ID NO:3, a CDR2 region comprising the amino acid sequence of SEQ ID NO:4 and a CDR3 region comprising the amino acid sequence of SEQ ID NO:5, and/or a light chain variable region comprising a CDR1 region comprising the amino acid sequence of SEQ ID NO:6, a CDR2 region comprising the amino acid sequence Gly-Ala-Ser and a CDR3 region comprising the amino acid sequence of SEQ ID NO:7.

An antibody or antigen binding fragment for use in the invention may comprise a light chain variable domain comprising an amino acid sequence of SEQ ID NO:10, or a sequence which has at least 90% amino acid sequence identity with SEQ ID NO:10, and/or a heavy chain variable domain comprising an amino acid sequence of SEQ ID NO:11, or a sequence which has at least 90% amino acid sequence identity with SEQ ID NO:1. Optionally, such an antibody may comprise a heavy chain comprising the amino acid sequence of SEQ ID NO:13, or a sequence which has at least 90% amino acid sequence identity to the amino acid sequence of SEQ ID NO:13, and/or a light chain comprising the amino acid sequence of SEQ ID NO:12, or a sequence which has at least 90% amino acid sequence identity to the amino acid sequence of SEQ ID NO:12, or an antigen binding fragment thereof.

In another embodiment, an antibody or antigen binding fragment for use in the present invention may comprise a heavy chain variable region comprising the amino acid sequence of SEQ ID NO:8, or a sequence which has at least 90% amino acid sequence identity to the amino acid sequence of SEQ ID NO:8, and/or a light chain variable region comprising the amino acid sequence of SEQ ID NO:9, or a sequence which has at least 90% amino acid sequence identity to the amino acid sequence of SEQ ID NO:9.

Antibodies and antibody fragments thereof for use in the present invention may be humanised. For example, an antibody for use in the invention may be a humanised version of anti-TLR2 antibody T2.5, or an antigen binding fragment thereof. A particular example of a humanised antibody which may be used in the present invention is OPN-305, or an antigen binding fragment thereof.

As described in the examples, the inventors have demonstrated that in ovarian tumours from subjects treated with a TLR2 antagonist, such as OPN-305, the expression of the mesenchymal gene Twist1 , a known driver of chemoresistance and metastasis, is reduced.

Accordingly, in some embodiments of the invention, said TLR2 inhibitor decreases Twist1 expression.

Moreover, as a further aspect, the invention provides a method of inhibiting expression of Twist1 in ovarian cancer cells, said method comprising administration of a TLR2 inhibitor.

Moreover, the inventors' data further shows that by inhibiting TLR2 pathway, the capacity of the tumour microenvironment to recruit myeloid derived suppressor cells (MDSCs) is reduced. Accordingly, in certain embodiments, said TLR2 inhibitor decreases levels of tumour infiltrating myeloid derived suppressor cells (MDSCs).

Moreover, as a further aspect, the invention provides a method of inhibiting infiltration of myeloid derived suppressor cells (MDSCs) into ovarian tumours, said method comprising administration of a TLR2 inhibitor.

The invention also enables the identification of candidate agents which may be used in the treatment of ovarian cancer, such as the treatment of recurrent ovarian cancer.

Thus, as a further aspect, the present invention provides a screening method for the identification of antibodies or antigen binding fragments thereof for use in treatment or prevention of ovarian cancer, the method comprising the steps of:
(a) providing candidate antibodies or antigen binding fragments thereof having binding specificity for TLR2;
(b) contacting the candidate antibodies or antigen binding fragments thereof with TLR2; and
(c) identifying antibodies or antigen binding fragments thereof which antagonise TLR2 function;
wherein antagonism of TLR2 function is indicative of utility of the antibody or antigen binding fragment thereof in the treatment or prevention of ovarian cancer.

Optionally, the method further includes step (d):
(d) testing the ability of said antibodies or antigen binding fragments thereof to inhibit Twist1 expression in ovarian cancer cells;
wherein ability to inhibit Twist1 expression in ovarian cancer cells is further indicative of utility of the antibodies or antigen binding fragments thereof in the treatment or prevention of ovarian cancer.

Optionally, the method may comprise step (e):
(e) testing the ability of said antibodies or antigen binding fragments thereof to inhibit infiltration of myeloid derived suppressor cells (MDSCs) into ovarian tumours;
wherein ability to inhibit infiltration of myeloid derived suppressor cells (MDSCs) into ovarian tumours is further indicative of utility of the antibodies or antigen binding fragments thereof in the treatment or prevention of ovarian cancer.

In certain embodiments the method comprises the step of administering a second TLR2 antagonist to the subject. For example, a TLR2 antagonistic antibody may be administered to prevent the activation of TLR2 and an inhibitory nucleic acid may also be administered to inhibit the expression of TLR2.

In certain embodiments, the TLR2 antagonist, such as TLR2 antibody or antigen binding fragment thereof, is administered to the subject prior to, during or following the subject undergoing a surgical procedure, such as resection.

Methods of screening for an increase in myeloid infiltrate will be known to persons skilled in the art, including, for example, screening for markers such as F4/80 and CD11b using routine methods such as flow cytometry or immunohistochemistry.

### Brief Description of the Figures

The present invention will now be described with reference to the following examples which are provided for the purpose of illustration and are not intended to be construed as being limiting on the present invention wherein:
Figure 1 shows schematically the experimental groups employed in Example 1 and details the number of animals per group.
Figure 2 shows treatment schedule and frequency.
Figure 3 shows a graph illustrating tumour kinetics during Primary treatment. The kinetics of tumour growth is significantly decreased in Paclitaxel (Group 2) and Paclitaxel + OPN-305 (Group 3) compared to Control (Group 1), * p < 0.0001; however, the addition of OPN-305 to Paclitaxel (Group 3) did not significantly change tumour kinetics compared to Paclitaxel alone (Group 2), ** p = 0.5959, Two way ANOVA).
Figure 4 shows that OPN-305 added to Paclitaxel significantly decreases tumour kinetics. When followed through maintenance treatment, all groups that received OPN-305 (Groups 2, 3, 4) had significantly decreased tumour growth compared to Paclitaxel - Saline (Group 1), * p <0.001. When Groups 2, 3, and 4 are compared to each other, the timing of OPN-305 administration did not result in a significant difference in tumour growth (** p > 0.05, Two way ANOVA).
Figure 5 shows graphs illustrating the effect of OPN-305 given as maintenance treatment post-Paclitaxel: **A,** significantly decrease tumour growth (p = 0.0001); **B,** significantly decrease final tumour burden (p = 0.0082); and **C,** significantly prolong progression-free interval (p = 0.0407).
Figure 6 shows barcharts illustrating that OPN-305 decreases tumour-infiltrating MDSCs. mCherry negative cells were gated in the FSC^{low} region and analysed for staining with Gr1 and/or CD11b. **A,** percentage of FSC^{low}, mCherry⁻/Gr1⁺/CD11b⁺ MDSCs; **B,** percentage of FSC^{low}, mCherry⁻/Gr1⁻/CD11b⁺ macrophages

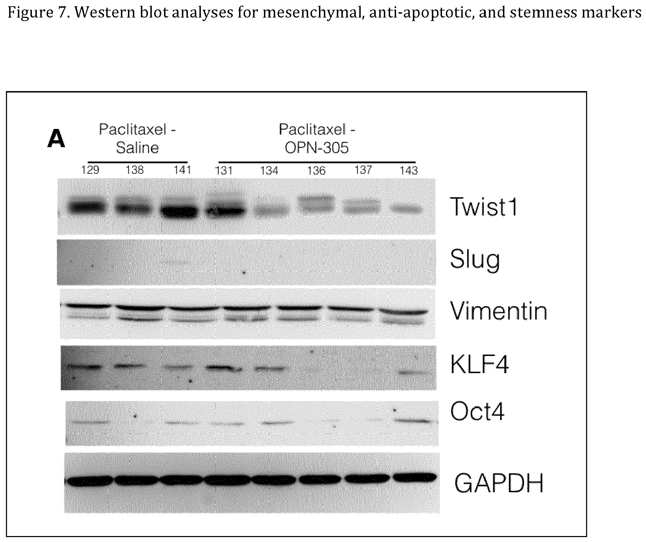

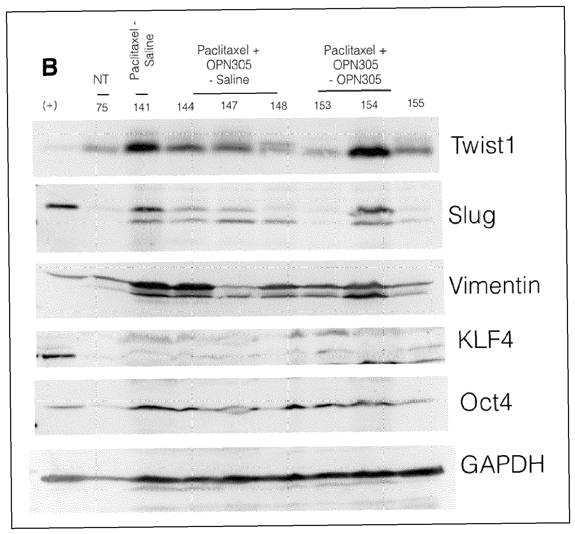

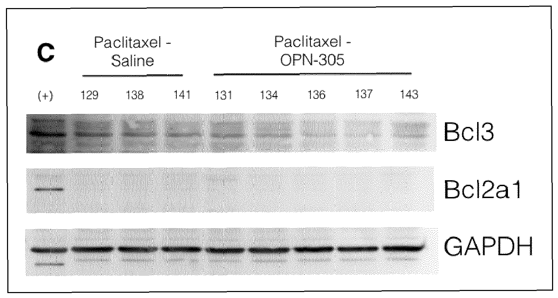

Figure 7 shows Western blot analyses for mesenchymal, anti-apoptotic, and stemness markers.

### Detailed Description of the Invention

The present inventor has shown that a TLR2 neutralising antibody may be used to treat ovarian cancer.

### Definitions

As herein defined, "Toll-like Receptor 2" may be also referred to as TLR2, TLR-2 or CD282. Typically, the TLR2 is human TLR2. Alternatively, the TLR2 is murine TLR2. In further embodiments, the TLR2 is a homologue or orthologue of human TLR2 which is derived from any mammal other than a human or mouse, for example, a cow or rat. In certain embodiments the TLR2 antibody is cross-reactive in that it mediates the suppression of TLR2 function in TLR2 derived from different species.

As herein defined, an agent is a "TLR2 antagonist" if the agent suppresses or blocks the activation or function of TLR2. The agent may inhibit or block binding of a ligand or binding compound to TLR2. This inhibition of TLR2 ligand binding may be achieved by a number of means, for example, through binding to the extracellular domain of TLR2 and partially or fully blocking the TLR2 ligand binding site, or by binding at a site other than the known TLR2 ligand binding site and inducing a conformational change upon binding which results in the TLR2 ligand binding site being altered in a manner which prevents TLR2 ligand binding or TLR2 activation. Further, the agent may inhibit or suppress intracellular signalling mediated by TLR2 following ligand binding and/or TLR2 activation. Intracellular signalling mediated following TLR2 activation and signalling results in the activation of transcription factors and the expression of genes which mediate a pro-inflammatory immune response. The agent may suppress or block TLR2 protein or gene expression, for example, by inhibiting the expression of a gene encoding a TLR2 protein.

As herein defined, the terms "blocks" and "blocking" when used in relation to TLR2 gene expression mean silencing the expression of at least one gene which results in the expression of the TLR2 protein. Gene silencing is the switching off of the expression of a gene by a mechanism other than genetic modification. Gene silencing can be mediated at the transcriptional level or at the post-transcriptional level. Transcriptional gene silencing can result in a gene being inaccessible to transcriptional machinery, and can be mediated, for example, by means of histone modifications. Post-transcriptional gene silencing results from the mRNA of a gene being destroyed, thus preventing an active gene product, such as a protein, in the present case the TLR2 protein.

The term "specifically binds" or "binding specificity" refers to the ability of a TLR2 binding compound (e.g. antibody or antigen binding fragment thereof) to bind to a target epitope present on TLR2 with a greater affinity than it binds to a non-target epitope. In certain embodiments specific binding refers to binding to a particular target epitope which is present on TLR2 with an affinity which is at least 10, 50, 100, 250, 500, or 1000 times greater than the affinity for a non-target epitope. Binding affinity may be determined by an affinity ELISA assay, a BIAcore assay, a kinetic method or by an equilibrium/solution method.

As herein defined, an "epitope" refers to a plurality of amino acid residues which are capable of being recognised by, and bound to by, a binding compound, such as a ligand, small molecule or antibody. Epitopes are generally comprised of chemically active surface groups and have specific three-dimensional structural characteristics, as well as specific charge characteristics which contribute to the three-dimensional structure of the epitope. The TLR2 antagonist antagonises the functional activity of TLR2 and as such binds to an epitope known as an inhibiting epitope or an inhibitory epitope. An "inhibiting" or "inhibitory" epitope means an epitope present on TLR2 that when bound by a binding compound, such as a small molecule or an antibody, results in the loss of biological activity of TLR2, for example due to the binding compound preventing the binding of TLR2 by a TLR2 agonist. The epitope that is present on TLR2, and which is bound by the binding compounds in order to antagonise TLR2 function, may comprise five or more amino acid residues.

The TLR2 antagonist for use in the invention may bind to a non-contiguous epitope. A "non-contiguous epitope" is an epitope that is comprised of a series of amino acid residues that are non-linear in alignment, such that the residues are spaced or grouped in a non-continuous manner along the length of a polypeptide sequence. The non-contiguous epitope described herein is a discontinuous scattered epitope wherein the residues which contribute to the epitope are provided in three or more groups of linear amino acid sequences arranged along the length of the TLR2 polypeptide.

As used herein, the term "subject" refers to an animal, preferably a mammal and in particular a human.

The term "consisting essentially of" as used herein means that the invention necessarily includes the listed items and is open to including unlisted items that do not materially affect the basic and novel properties of the invention.

The nomenclature used to describe the polypeptide constituents of the present invention follows the conventional practice wherein the amino group (N) is presented to the left and the carboxyl group to the right of each amino acid residue.

The expression "amino acid" as used herein is intended to include both natural and synthetic amino acids, and both D and L amino acids. A synthetic amino acid also encompasses chemically modified amino acids, including, but not limited to, salts, and amino acid derivatives such as amides. Amino acids can be modified by methylation, amidation, acetylation or substitution with other chemical groups which can change the circulating half-life without adversely affecting their biological activity.

The terms "peptide", "polypeptide" and "protein" are used herein interchangeably to describe a series of at least two amino acids covalently linked by peptide bonds or modified peptide bonds such as isosteres. No limitation is placed on the maximum number of amino acids which may comprise a peptide or protein. Furthermore, the term polypeptide extends to fragments, analogues and derivatives of a peptide, wherein said fragment, analogue or derivative retains the same biological functional activity as the peptide from which the fragment, derivative or analogue is derived

Furthermore the term "fusion protein" as used herein can also be taken to mean a fusion polypeptide, fusion peptide or the like, or may also be referred to as an immunoconjugate. The term "fusion protein" refers to a molecule in which two or more subunit molecules, typically polypeptides, are covalently or non-covalently linked.

As used herein, the term "therapeutically effective amount" means an amount which is sufficient to show benefit to the subject to whom the composition or TLR2 antagonist is administered, e.g. by reducing the severity of at least one symptom of ovarian cancer, by reducing tumour size, by suppressing tumour growth, by inhibiting angiogenesis, by suppressing metastasis, by delaying or preventing recurrence, by suppressing a T regulatory cell response and/or by promoting a Th1 response.

As used herein, the term "treatment" and associated terms such as "treat" and "treating" means the reduction of the progression or severity of ovarian cancer or at least one symptom thereof, e.g. by reducing tumour size, by suppressing tumour growth, by inhibiting angiogenesis, by suppressing metastasis, by delaying or preventing recurrence, by suppressing a T regulatory cell response and/or by promoting a Th1 response. The term "treatment" refers to any regimen that can benefit a subject. The treatment may be in respect of an existing ovarian cancer condition or may be prophylactic (preventative treatment). Treatment may include curative, alleviative or prophylactic effects. References herein to "therapeutic" and "prophylactic" treatments are to be considered in their broadest context. The term "therapeutic" does not necessarily imply that the subject is treated until total recovery. Similarly, "prophylactic" does not necessarily mean that the subject will not eventually contract a disease condition.

Unless otherwise defined, all technical and scientific terms used herein have the meaning commonly understood by a person who is skilled in the art in the field of the present invention.

Throughout the specification, unless the context demands otherwise, the terms "comprise" or "include", or variations such as "comprises" or "comprising", "includes" or "including" will be understood to imply the inclusion of a stated integer or group of integers, but not the exclusion of any other integer or group of integers.

As used herein, terms such as "a", "an" and "the" include singular and plural referents unless the context clearly demands otherwise. Thus, for example, reference to "an active agent" or "a pharmacologically active agent" includes a single active agent as well as two or more different active agents in combination, while references to "a carrier" includes mixtures of two or more carriers as well as a single carrier, and the like.

### TLR2 Antagonists

Any suitable TLR2 antagonist may be used in the present invention. In certain embodiments the TLR2 antagonist is selected from the group consisting of a protein, a peptide, an antibody or an antigen binding fragment thereof, a peptidomimetic, a nucleic acid, a carbohydrate, a lipid and a small molecule.

For example, the TLR2 antagonist may be an antibody or binding fragment thereof. In an alternative embodiment, the TLR2 antagonist is a nucleic acid inhibitor of TLR2.

### Antibodies

The TLR2 antagonist may be an antibody or an antigen binding fragment thereof. An "antibody" is an immunoglobulin, whether natural or partly or wholly synthetically produced. The term also covers any polypeptide, protein or peptide having a binding domain that is, or is homologous to, an antibody binding domain. These can be derived from natural sources, or they may be partly or wholly synthetically produced. Examples of antibodies are the immunoglobulin isotypes and their isotypic subclasses and fragments which comprise an antigen binding domain such as Fab, scFv, Fv, dAb or Fd, and a bi-specific antibody.

In certain embodiments the antibody may be a camelid antibody, in particular a camelid heavy chain antibody. Further, the antibody fragment may be a domain antibody or a nanobody derived from a camelid heavy chain antibody. In certain embodiments the antibody may be a shark antibody or a shark derived antibody.

In certain embodiments the antibody is an "isolated antibody", this meaning that the antibody is (1) free of at least some proteins with which it would normally be found, (2) is essentially free of other proteins from the same source, e.g., from the same species, (3) is expressed by a cell from a different species, or (4) does not occur in nature.

As antibodies can be modified in a number of ways, the term "antibody" should be construed as covering any binding member or substance having a binding domain with the required specificity. The antibody of the invention may be a monoclonal antibody, or a fragment, derivative, functional equivalent or homologue thereof. The term includes any polypeptide comprising an immunoglobulin binding domain, whether natural or wholly or partially synthetic. Chimeric molecules comprising an immunoglobulin binding domain, or equivalent, fused to another polypeptide are therefore included.

The constant region of the antibody may be of any suitable immunoglobulin subtype. In certain embodiments the subtype of the antibody may be of the class IgA, IgM, IgD and IgE where a human immunoglobulin molecule is used. Such an antibody may further belong to any subclass, e.g. IgG1, IgG2a, IgG2b, IgG3 and IgG4.

Fragments of a whole antibody can perform the function of antigen binding. Examples of such binding fragments are a Fab fragment comprising or consisting of the VL, VH, CL and CH1 antibody domains; an Fv fragment consisting of the VL and VH domains of a single antibody; a F(ab')2 fragment; a bivalent fragment comprising two linked Fab fragments; a single chain Fv molecule (scFv), wherein a VH domain and a VL domain are linked by a peptide linker which allows the two domains to associate to form an antigen binding site; and a bi-specific antibody, which may be multivalent or multispecific fragments constructed by gene fusion.

A fragment of an antibody for use in the present invention generally means a stretch of amino acid residues of at least 5 to 7 contiguous amino acids, often at least about 7 to 9 contiguous amino acids, typically at least about 9 to 13 contiguous amino acids, more preferably at least about 20 to 30 or more contiguous amino acids and most preferably at least about 30 to 40 or more consecutive amino acids.

A "derivative" of such an antibody or of a fragment of a TLR2 specific antibody means an antibody or polypeptide modified by varying the amino acid sequence of the protein, e.g. by manipulation of the nucleic acid encoding the protein or by altering the protein itself. Such derivatives of the natural amino acid sequence may involve insertion, addition, deletion and/or substitution of one or more amino acids, preferably while providing a peptide having TLR2 binding activity. Preferably such derivatives involve the insertion, addition, deletion and/or substitution of 25 or fewer amino acids, more preferably of 15 or fewer, even more preferably of 10 or fewer, more preferably still of 4 or fewer and most preferably of 1 or 2 amino acids only.

In certain embodiments humanized antibodies may be used. A humanised antibody may be a modified antibody having the hypervariable region of a TLR2 specific antibody and the constant region of a human antibody. Thus the binding member may comprise a human constant region. The variable region other than the hypervariable region may also be derived from the variable region of a human antibody and/or may also be derived from a TLR2 specific antibody. In other cases, the entire variable region may be derived from a murine monoclonal TLR2 specific antibody and the antibody is said to be chimerised.

It is possible to take monoclonal and other antibodies and use techniques of recombinant DNA technology to produce other antibodies or chimeric molecules which retain the specificity of the original antibody. Such techniques may involve introducing DNA encoding the immunoglobulin variable region, or the complementarity determining regions (CDRs), of an antibody to the constant regions, or constant regions plus framework regions, of a different immunoglobulin. A hybridoma or other cell producing an antibody may be subject to genetic mutation or other changes, which may or may not alter the binding specificity of antibodies produced.

Typically a TLR2 antibody or antigen binding fragment thereof for use in the present invention has binding specificity to TLR2, typically human TLR2. Typically the TLR2 antibody or antigen binding fragment thereof has binding specificity to an epitope comprising amino acid residues of the extracellular domain of TLR2. The extracellular domain of the human form of TLR2 comprises 587 amino acid residues, specifically amino acids 1-587 of the 784 amino acid full length human TLR2 sequence shown as SEQ ID NO:1 and defined as Genbank Accession Number AAC 34133 (URL www.ncbi.nlm.nih.gov). Typically the antibody or antigen binding fragment thereof binds to the ligand binding region of TLR2. Typically the antibody or antigen binding fragment thereof inhibits or prevents dimerization of TLR2 with Toll-like Receptor 1 (TLR1) and/or Toll-like Receptor 6 (TLR6) by covering the interaction surface between TLR2 and TLR1 and/or TLR6. Typically the antibody or antigen binding fragment thereof suppresses the function of TLR2 irrespective of whether TLR2 forms a heterodimer with TLR1, Toll-like Receptor 4 (TLR4), TLR6 or Toll-like Receptor 10 (TLR10). Typically the binding region/interaction surface is located within leucine-rich repeat (LRR) regions 11 to 14 of TLR2. In certain embodiments the TLR2 antibody or antigen binding fragment thereof has binding specificity to an epitope comprising, consisting of or consisting essentially of LRR regions 11 to 14 of TLR2, or a sequence which has at least 85%, 90% or 95% sequence identity thereto.

In certain embodiments the TLR2 antibody or antigen binding fragment thereof binds to a non-continuous epitope comprising, consisting of or consisting essentially of amino acid residues His318 (H, histidine), Pro320 (P, proline), Arg321 (R, arginine) or Gln321 (Q, glutamine), Tyr323 (Y, tyrosine), Lys347 (K, lysine), Phe349 (F, phenylalanine), Leu371 (L, leucine), Glu375 (E, glutamic acid), Tyr376 (Y, tyrosine), and His398 (H, histidine) of SEQ ID NO: 1 or SEQ ID NO:2.

In certain embodiments the TLR2 antibody or antigen binding fragment thereof binds to a non-continuous epitope comprising, consisting of or consisting essentially of amino acid residues His318 (H, histidine), Pro320 (P, proline), Arg321 (R, arginine), Tyr323 (Y, tyrosine), Lys347 (K, lysine), Phe349 (F, phenylalanine), Leu371 (L, leucine), Glu375 (E, glutamic acid), Tyr376 (Y, tyrosine), and His398 (H, histidine) of SEQ ID NO: 1.
SEQ ID NO:1 (human TLR2)

In certain embodiments the TLR2 antibody or antigen binding fragment thereof binds to a non-continuous epitope comprising, consisting of or consisting essentially of amino acid residues His318 (H, histidine), Pro320 (P, proline), Gln321 (Q, glutamine), Tyr323 (Y, tyrosine), Lys347 (K, lysine), Phe349 (F, phenylalanine), Leu371 (L, leucine), Glu375 (E, glutamic acid), Tyr376 (Y, tyrosine), and His398 (H, histidine) of murine TLR2 (SEQ ID NO: 2). SEQ ID NO:2 shows the amino acid murine TLR2 sequence defined as Genbank Accession Number NP_036035 (*Mus musculus*).
SEQ ID NO:2 (murine TLR2)

MLRALWLFWI LVAITVLFSK RCSAQESLSC DASGVCDGRS RSFTSIPSGL TAAMKSLDLS
FNKITYIGHG DLRACANLQV LMLKSSRINT IEGDAFYSLG SLEHLDLSDN HLSSLSSSWF
GPLSSLKYLN LMGNPYQTLG VTSLFPNLTN LQTLRIGNVE TFSEIRRIDF AGLTSLNELE
IKALSLRNYQ SQSLKSIRDI HHLTLHLSES AFLLEIFADI LSSVRYLELR DTNLARFQFS
PLPVDEVSSP MKKLAFRGSV LTDESFNELL KLLRYILELS EVEFDDCTLN GLGDFNPSES
DVVSELGKVE TVTIRRLHIP QFYLFYDLST VYSLLEKVKR ITVENSKVFL VPCSFSQHLK
SLEFLDLSEN LMVEEYLKNS ACKGAWPSLQ TLVLSQNHLR SMQKTGEILL TLKNLTSLDI
SRNTFHPMPD SCQWPEKMRF LNLSSTGIRV VKTCIPQTLE VLDVSNNNLD SFSLFLPRLQ
ELYISRNKLK TLPDASLFPV LLVMKIRENA VSTFSKDQLG SFPKLETLEA GDNHFVCSCE
LLSFTMETPA LAQILVDWPD SYLCDSPPRL HGHRLQDARP SVLECHQAAL VSGVCCALLL
LILLVGALCH HFHGLWYLRM MWAWLQAKRK PKKAPCRDVC YDAFVSYSEQ
DSHWVENLMV QQLENSDPPF KLCLHKRDFV PGKWIIDNII DSIEKSHKTV FVLSENFVRS
EWCKYELDFS HFRLFDENND AAILVLLEPI ERKAIPQRFC KLRKIMNTKT YLEWPLDEGQ
QEVFWVNLRT AIKS

The above identified epitope in humans or mice is a functional epitope for receptor dimerization or inhibition thereof. Typically the epitope is bound by the TLR2 antagonistic antibodies T2.5 and OPN-305. Binding of the identified epitope by an antagonistic antibody or an antigen binding fragment thereof results in antagonism of TLR2 biological function, in particular activation and signalling. In particular, binding by the antibody or antigen binding fragment thereof serves to inhibit activation of the TLR2 receptor, irrespective of whether a TLR2 heterodimer is formed with another TLR, such as TLR1, TLR6, TLR4 or TLR10. Furthermore, antibodies binding to this epitope have been shown to cross-react with TLR2 from human, pig and monkey, indicating this to be a critical epitope in a highly conserved region of TLR2.

In certain embodiments the antibody is selected from the group consisting of a human antibody, a humanised antibody, a chimeric antibody, a monoclonal antibody, a polyclonal antibody, a synthetic antibody, a camelid antibody, a shark antibody and an *in-vitro* antibody. In certain embodiments an antigen binding fragment may be used. The antigen binding fragment may be derived from any of the aforementioned antibodies. In certain embodiments the antigen binding fragment is selected from the group consisting of a Fab fragment, a scFv fragment, a Fv fragment and a dAb fragment. In certain embodiments the antibody comprises two complete heavy chains and two complete light chains, or an antigen binding fragment thereof. In certain embodiments the antibody is of the isotype IgG, IgA, IgE or IgM, or an antigen binding fragment thereof. In certain embodiments where the antibody is of the isotype IgG, the antibody may be of the subtype IgG1, IgG2 or IgG3, or an antigen binding fragment thereof. In certain embodiments the antibody is of the subtype IgG4, or an antigen binding fragment thereof. The antibody or antigen binding fragment may bind to an inhibitory epitope present on TLR2 with a dissociation constant (Kd) of from about 10⁻⁷M to about 10⁻¹¹M. More particularly the antibody or antigen binding fragment thereof may bind to mammalian (e.g. human or murine) TLR2 with a K_{D} of 4 x 10⁻⁸ M or less. Even more particularly the antibody or antigen binding fragment thereof may bind to human TLR2 with a K_{D} of 3 x 10⁻⁸ M or less. In certain embodiments the antibody is an isolated antibody or an antigen binding fragment thereof.

In certain embodiments the antibody or antigen binding fragment comprises a heavy chain variable region comprising the heavy chain variable region complementarity regions of the murine IgG1 anti-TLR2 antibody derived from hybridoma clone T2.5 (HyCult Biotechnology b.v., Cell Sciences, Canton, USA: catalogue number 1054) and/or a light chain variable region comprising the light chain variable region complementarity regions of the murine IgG1 anti-TLR2 antibody derived from hybridoma clone T2.5 (HyCult Biotechnology b.v., Cell Sciences, Canton, USA: catalogue number 1054). In certain embodiments the antibody or antigen binding fragment comprises a heavy chain variable region comprising a complementarity determining region 1 (CDR1) region comprising the amino acid sequence Gly-Phe-Thr-Phe-Thr-Thr-Tyr-Gly (SEQ ID NO:3), a CDR2 region comprising the amino acid sequence Ile-Tyr-Pro-Arg-Asp-Gly-Ser-Thr (SEQ ID NO:4) and a CDR3 region comprising the amino acid sequence Ala-Arg-Leu-Thr-Gly-Gly-Thr-Phe-Leu-Asp-Tyr (SEQ ID NO:5), and/or a light chain variable region comprising a CDR1 region comprising the amino acid sequence Glu-Ser-Val-Glu-Tyr-Tyr-Gly-Thr-Ser-Leu (SEQ ID NO:6), a CDR2 region comprising the amino acid sequence Gly-Ala-Ser and a CDR3 region comprising the amino acid sequence Gln-Gln-Ser-Arg-Lys-Leu-Pro-Trp-Thr (SEQ ID NO:7).

In certain embodiments the antibody or antigen binding fragment comprises a heavy chain variable region of the murine IgG1 anti-TLR2 antibody derived from hybridoma clone T2.5 (HyCult Biotechnology b.v., Cell Sciences, Canton, USA: catalogue number 1054) and/or a light chain variable region of the murine IgG1 anti-TLR2 antibody derived from hybridoma clone T2.5 (HyCult Biotechnology b.v., Cell Sciences, Canton, USA: catalogue number 1054). In certain embodiments the heavy chain variable region comprises or consists of the amino acid sequence as depicted in SEQ ID NO:8, or a sequence which has at least 85%, 90%, 95%, 96%, 97%, 98% or 99% amino acid sequence identity over a length of at least 20, and up to all, amino acids of the amino acid sequence of SEQ ID NO:8, and/or the light chain variable region comprises or consists of the amino acid sequence as depicted in SEQ ID NO:9, or a sequence which has at least 85%, 90%, 95%, 96%, 97%, 98% or 99% amino acid sequence identity over a length of at least 20, and up to all, amino acids of the amino acid sequence of SEQ ID NO:9.

In certain embodiments the antibody or antigen binding fragment comprises a heavy chain variable region of a humanised version of the murine IgG1 anti-TLR2 antibody derived from hybridoma clone T2.5 (HyCult Biotechnology b.v., Cell Sciences, Canton, USA: catalogue number 1054) and/or a light chain variable region of a humanised version the murine IgG1 anti-TLR2 antibody derived from hybridoma clone T2.5 (HyCult Biotechnology b.v., Cell Sciences, Canton, USA: catalogue number 1054). In certain embodiments the antibody or antigen binding fragment comprises a heavy chain variable region of the OPN-305 antibody as described in WO2011/003925 and/or a light chain variable region of the OPN-305 antibody as described in WO2011/003925. In certain embodiments the antibody or antigen binding fragment comprises a light chain variable domain comprising or consisting of an amino acid sequence of SEQ ID NO:10, or a sequence which has at least 85%, 90%, 95%, 96%, 97%, 98% or 99% amino acid sequence identity over a length of at least 20, and up to all, amino acids of the amino acid sequence of SEQ ID NO:10, and/or a heavy chain variable domain comprising or consisting of an amino acid sequence of SEQ ID NO:11, or a sequence which has at least 85%, 90%, 95%, 96%, 97%, 98% or 99% amino acid sequence identity over a length of at least 20, and up to all, amino acids of the amino acid sequence of SEQ ID NO:11. Typically, the antibody or antigen binding fragment specifically binds to TLR2 and does not bind to CD32. Typically the antibody or antigen binding fragment thereof mediates TLR2 antagonism independently of binding of the antibody or antigen binding fragment thereof to TLR2.

In certain embodiments the antibody or antigen binding fragment comprises a heavy chain of a humanised version of the murine IgG1 anti-TLR2 antibody derived from hybridoma clone T2.5 (HyCult Biotechnology b.v., Cell Sciences, Canton, USA: catalogue number 1054) and/or a light chain of a humanised version the murine IgG1 anti-TLR2 antibody derived from hybridoma clone T2.5 (HyCult Biotechnology b.v., Cell Sciences, Canton, USA: catalogue number 1054), or an antigen binding fragment thereof. In certain embodiments the antibody or antigen binding fragment comprises a heavy chain of the OPN-305 antibody as described in WO2011/003925 and/or a light chain of the OPN-305 antibody as described in WO2011/003925. In certain embodiments the light chain comprises or consists of the amino acid sequence of SEQ ID NO: 12, or a sequence which has at least 85%, 90%, 95%, 96%, 97%, 98% or 99% amino acid sequence identity over a length of at least 20, and up to all, amino acids of the amino acid sequence of SEQ ID NO:12, and/or the heavy chain comprises the amino acid sequence of SEQ ID NO:13, or a sequence which has at least 85%, 90%, 95%, 96%, 97%, 98% or 99% amino acid sequence identity over a length of at least 20, and up to all, amino acids of the amino acid sequence of SEQ ID NO:13. Typically, the antibody or antigen binding fragment is a fully humanised antibody. In certain embodiments the variable domain of the heavy chain is joined to a constant domain derived from an antibody of the subclass immunoglobulin G, isotype 4. In certain embodiments amino acid residue 241 of a hinge region of the heavy chain is substituted from a serine residue to a proline residue. In certain embodiments the antibody or antigen binding fragment binds to mammalian Toll-like Receptor 2 with a K_{D} of 1x10⁻⁸ M or less. In certain embodiments the antibody or antigen binding fragment binds to mammalian (e.g. human or murine) TLR2 with a K_{D} of 4 x 10⁻⁸ M or less. In certain embodiments the antibody or antigen binding fragment binds to human TLR2 with a K_{D} of 3 x 10⁻⁸ M or less.

In certain embodiments the antibody or antigen binding fragment is derived from hybridoma clone T2.5 (HyCult Biotechnology b.v., Cell Sciences, Canton, USA: catalogue number 1054), or is a humanised version thereof. In certain embodiments the antibody is a murine IgG1 anti-TLR2 antibody derived from hybridoma clone T2.5 (HyCult Biotechnology b.v., Cell Sciences, Canton, USA: catalogue number 1054), or a humanised version or antigen binding fragment thereof. In certain embodiments the humanised version thereof is the OPN-305 antibody, as described in WO2011/003925.

In certain embodiments the TLR2 antibody or antigen binding fragment thereof is targeted to the ovaries or TLR2 expressed on ovarian cells or tissue. In certain embodiments the TLR2 antibody or antigen binding fragment thereof is targeted specifically to tumour cells. Targeting may be by any suitable means known to the person skilled in the art, such as localised delivery, the use of a delivery vector or a targeting means, such as an antibody which has binding specificity for a cell surface target expressed on ovarian cells and/or tumour tissue. The targeting of the TLR2 antibody or antigen binding fragment thereof in this way is advantageous as systemic administration of the TLR2 antibody or antigen binding fragment thereof may result in global immunosuppression of the TLR2 receptor and accordingly TLR2 mediated signalling which may be undesirable in some instances. Targeting of the TLR2 antibody or antigen binding fragment thereof may be provided through the formation of a fusion protein, wherein said fusion protein is comprised of a TLR2 antibody or antigen binding fragment thereof conjoined to a secondary peptide, typically the Fc receptor binding protein derived from the heavy chain of an immunoglobulin, typically a human immunoglobulin. The Fc domain has been extensively used to prolong the circulatory half-life of therapeutic proteins.

In certain embodiments the TLR2 antibody or antigen binding fragment thereof binds to the epitope present on the extracellular domain of TLR2 with an affinity constant (Ka) of at least 10⁻⁶M.

In certain embodiments the therapeutically effective amount comprises the antibody in a range chosen from 1 µg/kg to 20 mg/kg, 1 g/kg to 10 mg/kg, 1 µg/kg to 1 mg/kg, 10 µg/kg to 1 mg/kg, 10 µg/kg to 100 pg/kg and 500 pg/kg to 1 mg/kg.

Also suitable for use in the present invention are TLR2 antagonists comprising a nucleic acid encoding an antibody or antigen binding fragment as described above, or a vector comprising said nucleic acid.

### Production of Antibodies

The antibodies provided for use in the present invention may be provided by a number of techniques. For example, a combinatorial screening technique such as a phage display-based biopanning assay may be used to in order to identify amino acid sequences which have binding specificity to TLR2, in particular the binding epitopes described above. Such phage display biopanning techniques involve the use of phage display libraries, which are utilised in methods which identify suitable epitope binding ligands in a procedure which mimics immune selection, through the display of antibody binding fragments on the surface of filamentous bacteria. Phage with specific binding activity are selected. The selected phage can thereafter be used in the production of chimeric, CDR-grafted, humanised or human antibodies. Antibodies can be tested for their ability to antagonise TLR2 function using methods known in the art.

In further embodiments the antibody is a monoclonal antibody, which may be produced using any suitable method which produces antibody molecules by continuous cell lines in culture. Chimeric antibodies or CDR-grafted antibodies are further provided for use in the present invention. In certain embodiments, the antibodies for use in the invention may be produced by the expression of recombinant DNA in a host cell.

In certain embodiments the monoclonal antibodies may be human antibodies, produced using transgenic animals, for example, transgenic mice, which have been genetically modified to delete or suppress the expression of endogenous murine immunoglobulin genes, with loci encoding for human heavy and light chains being expressed in preference, this resulting in the production of fully human antibodies.

In certain embodiments the TLR2 antagonist is a binding fragment which is derived from an antibody, for example, an antibody binding fragment, such as a Fab, F(ab')2, Fv or a single chain Fv (scFV).

In certain embodiments the TLR2 antibody comprises a polyclonal antibody, a chimeric antibody, a synthesized or synthetic antibody, a fusion protein or fragment thereof, a natural or synthetic chemical compound or a peptidomimetic.

Antibodies or antigen fragments for use in the present invention may also be generated wholly or partly by chemical synthesis. The antibodies can be readily prepared according to well-established, standard liquid or, preferably, solid-phase peptide synthesis methods, general descriptions of which are broadly available and are well known by the person skilled in the art. Further, they may be prepared in solution, by the liquid phase method or by any combination of solid-phase, liquid phase and solution chemistry.

Another convenient way of producing antibodies or antibody fragments suitable for use in the present invention is to express nucleic acid encoding them by use of nucleic acid in an expression system.

Recombinant DNA technology may be used to improve the antibodies for use in the invention. Thus, chimeric antibodies may be constructed in order to decrease the immunogenicity thereof in diagnostic or therapeutic applications. Moreover, immunogenicity within, for example, a transgenic organism such as a pig, may be minimised, by altering the antibodies by CDR grafting in a technique analogous to humanising antibodies. In order to reduce immunogenicity within a human recipient, the invention may employ recombinant nucleic acids comprising an insert coding for a heavy chain variable domain of an antibody fused to a human constant domain. Likewise the invention concerns recombinant DNAs comprising an insert coding for a light chain variable domain of an antibody fused to a human constant domain kappa or lambda region.

Antibodies may moreover be generated by mutagenesis of antibody genes to produce 5 artificial repertoires of antibodies. This technique allows the preparation of antibody libraries. Antibody libraries are also available commercially. Hence, the present invention advantageously employs artificial repertoires of immunoglobulins, preferably artificial scFv repertoires, as an immunoglobulin source in order to identify binding molecules which have specificity for the epitope described above.

### Antibody selection systems

Immunoglobulins which are able to bind to and antagonise TLR2 and which accordingly may be used in the methods of the invention can be identified using any technique known to the skilled person. Such immunoglobulins may be conveniently isolated from libraries comprising artificial repertoires of immunoglobulin polypeptides. A "repertoire" refers to a set of molecules generated by random, semi-random or directed variation of one or more template molecules, at the nucleic acid level, in order to provide a multiplicity of binding specificities. Methods for generating repertoires are well characterised in the art.

Any library selection system may be used in conjunction with the invention. Selection protocols for isolating desired members of large libraries are known in the art, as typified by phage display techniques. Such systems, in which diverse peptide sequences are displayed on the surface of filamentous bacteriophage, have proven useful for creating libraries of antibody fragments (and the nucleotide sequences that encode them) for the *in-vitro* selection and amplification of specific antibody fragments that bind a target antigen. The nucleotide sequences encoding the VH and VL regions are linked to gene fragments which encode leader signals that direct them to the periplasmic space of *E. coli* and as a result the resultant antibody fragments are displayed on the surface of the bacteriophage, typically as fusions to bacteriophage coat proteins (for example pIII or pVIII). Alternatively, antibody fragments are displayed externally on lambda phage capsids (phage bodies). An advantage of phage-based display systems is that, because they are biological systems, selected library members can be amplified simply by growing the phage containing the selected library member in bacterial cells. Furthermore, since the nucleotide sequence that encodes the polypeptide library member is contained on a phage or phagemid vector, sequencing, expression and subsequent genetic manipulation is relatively straight forward.

Methods for the construction of bacteriophage antibody display libraries and lambda phage expression libraries are well known in the art.

An alternative to the use of phage or other cloned libraries is to use nucleic acid, preferably RNA, derived from the B cells of an animal which has been immunised with the selected target, e.g. the TLR2 epitope described above.

Isolation of V-region and C-region mRNA permits antibody fragments, such as Fab or Fv, to be expressed intracellularly. Briefly, RNA is isolated from the B cells of an immunised animal, for example from the spleen of an immunised mouse or the circulating B cells of a llama, and PCR primers used to amplify VH and VL cDNA selectively from the RNA pool. The VH and VL sequences thus obtained are joined to make scFv antibodies. PCR primer sequences may be based on published VH and VL sequences.

A method for producing polypeptides comprises culturing host cells transformed with a recombinant expression vector encoding a polypeptide under conditions that promote expression of the polypeptide, then recovering the expressed polypeptides from the culture. The person skilled in the art will recognise that the procedure for purifying the expressed polypeptides will vary according to such factors as the type of host cells employed, and whether the polypeptide is intracellular, membrane-bound or a soluble form that is secreted from the host cell.

Any suitable expression system may be employed. The vectors include a DNA encoding a polypeptide or fragment of the invention, operably linked to suitable transcriptional or translational regulatory nucleotide sequences, such as those derived from a mammalian, avian, microbial, viral, bacterial, or insect gene. Nucleotide sequences are operably linked when the regulatory sequence functionally relates to the DNA sequence.

Thus, a promoter nucleotide sequence is operably linked to a DNA sequence if the promoter nucleotide sequence controls the transcription of the DNA sequence. An origin of replication that confers the ability to replicate in the desired (*E.coli*) host cells, and a selection gene by which transformants are identified, are generally incorporated into the expression vector.

In addition, a sequence encoding an appropriate signal peptide (native or heterologous) can be incorporated into expression vectors. A DNA sequence for a signal peptide (secretory leader) may be fused in frame to the nucleic acid sequence of the invention so that the DNA is initially transcribed, and the mRNA translated, into a fusion protein comprising the signal peptide. A signal peptide that is functional in the intended host cells promotes extracellular secretion of the polypeptide. The signal peptide is cleaved from the polypeptide during translation, but allows secretion of polypeptide from the cell.

Suitable host cells for expression of polypeptides include higher eukaryotic cells and yeast. Prokaryotic systems are also suitable. Mammalian cells, and in particular CHO cells are particularly preferred for use as host cells.

### Nucleic acid

Also contemplated for use in the present invention are TLR2 antagonists comprising an inhibitory nucleic acid which inhibits the expression of at least one nucleic acid which encodes TLR2 protein. The TLR2 antagonist may be selected from the group consisting of anti-sense oligonucleotides, triple helix molecules, anti-sense DNA, anti-sense RNA, ribozyme, iRNA, miRNA, siRNA and shRNA. Accordingly, a therapeutically effective amount of an inhibitory nucleic acid, such as an RNAi (RNA interference) agent, for example an interfering ribonucleic acid (such as siRNA or shRNA) or a transcription template thereof, such as a DNA encoding an shRNA may be administered to a subject in order to block the expression of the TLR2 protein. The inhibitory nucleic acid may be an antisense RNA molecule. Antisense causes suppression of gene expression and involves single stranded RNA fragments which physically bind to mRNA, thus blocking mRNA translation. Techniques for the preparation of inhibitory nucleic acids are known to persons skilled in the art.

Nucleic acid for use in accordance with the present invention may comprise DNA or RNA and may be wholly or partially synthetic. In a preferred aspect, nucleic acid for use in the invention codes for antibodies or antibody fragments of the invention as defined above. The skilled person will be able to determine substitutions, deletions and/or additions to such nucleic acids which will still provide an antibody or antibody fragment of the present invention.

Nucleic acid sequences encoding antibodies or antibody fragments for use with the present invention can be readily prepared by the skilled person. These techniques include (i) the use of the polymerase chain reaction (PCR) to amplify samples of such nucleic acid, e.g. from genomic sources, (ii) chemical synthesis, or (iii) preparing cDNA sequences. DNA encoding antibody fragments may be generated and used in any suitable way known to those of skill in the art, including by taking encoding DNA, identifying suitable restriction enzyme recognition sites either side of the portion to be expressed, and cutting out said portion from the DNA. The portion may then be operably linked to a suitable promoter in a standard commercially available expression system. Another recombinant approach is to amplify the relevant portion of the DNA with suitable PCR primers. Modifications to the sequences can be made, e.g. using site directed mutagenesis, to lead to the expression of modified peptide or to take account of codon preferences in the host cells used to express the nucleic acid.

The nucleic acid may be comprised as constructs in the form of a plasmid, vector, transcription or expression cassette which comprises at least one nucleic acid as described above. The construct may be comprised within a recombinant host cell which comprises one or more constructs as above. Expression may conveniently be achieved by culturing under appropriate conditions recombinant host cells containing the nucleic acid. Following production by expression the antibody or antibody fragments may be isolated and/or purified using any suitable technique, then used as appropriate.

Systems for cloning and expression of a polypeptide in a variety of different host cells are well known. Suitable host cells include bacteria, mammalian cells, yeast, insect and baculovirus systems. Mammalian cell lines available in the art for expression of a heterologous polypeptide include Chinese hamster ovary (CHO) cells, HeLa cells, baby hamster kidney cells, NS0 mouse myeloma cells. A common, preferred bacterial host is *E. coli.* The expression of antibodies and antibody fragments in prokaryotic cells such as *E. coli* is well established in the art. Expression in eukaryotic cells in culture is also available to those skilled in the art as an option for production of a binding member. General techniques for the production of antibodies are well known to the person skilled in the field.

In certain embodiments of the invention, recombinant nucleic acids comprising an insert coding for a heavy chain variable domain and/or for a light chain variable domain of antibodies are provided. By definition such nucleic acids comprise coding single stranded nucleic acids, double stranded nucleic acids consisting of said coding nucleic acids and of complementary nucleic acids thereto, or these complementary (single stranded) nucleic acids themselves.

Furthermore, nucleic acids encoding a heavy chain variable domain and/or a light chain variable domain of antibodies can be enzymatically or chemically synthesised nucleic acids having the authentic sequence coding for a naturally-occurring heavy chain variable domain and/or for the light chain variable domain, or a mutant thereof.

### Soluble TLR2

The inventor has further identified that suppression of the function of TLR2 can be achieved by means of reducing the amount of ligand which is available to bind to and activate membrane bound TLR2. A reduction in the amount of ligand which is available to bind membrane bound TLR2 results in a downregulation of TLR2-mediated signalling. Accordingly, the TLR2 antagonist for use in the invention may be a soluble form of recombinant TLR2 (sTLR2), or a functional fragment thereof. The soluble form of TLR2 competes with the membrane bound form of TLR2 for TLR2 specific binding ligands. This competitive binding results in the soluble form of TLR2 effectively "mopping up" available TLR2 ligand with an associated reduction in the binding and activation of membrane bound TLR2.

The soluble form of TLR2 may be prepared by a recombinant technique. A soluble form of TLR2 typically comprises the extracellular domain of TLR2 only, and hence the intracellular and transmembrane domains of TLR2 as defined in Genbank Accession Number AAC 34133 (SEQ ID NO:1) are absent. The soluble form of TLR2 may comprise amino acids 1 to 587 of SEQ ID NO:1. The soluble TLR2 sequence may be modified by means of the addition, deletion or substitution of one or more amino acid residues. Accordingly, the soluble form of TLR2 may be derived from a truncated form of the full length membrane bound TLR2 amino acid sequence or, in addition to a deletion and/or substitution of the amino acids residues relating to the intracellular and/or transmembrane domains in the membrane bound form of TLR2, a deletion and/or substitution may further be made to the amino acid residues of the extracellular domain. Any such truncation or deletion and/or substitution of the amino acid residues of the extracellular domain of the TLR2 may be made so long as the modified form of TLR2 is soluble and capable of binding a ligand which can bind to at least one epitope present on the membrane bound form of TLR2.

### Chemotherapeutic Agents

In certain embodiments the TLR2 antagonists are for use in combination treatment with a therapeutically effective amount of a secondary therapeutic compound, such as a chemotherapeutic agent, suitable for use in the treatment or prevention of ovarian cancer. The inventor has shown that combination therapies comprising a TLR2 antagonist as described above and a chemotherapeutic agent show significant benefits over chemotherapeutic treatment alone.

Any suitable chemotherapeutic agent may be used. For example, a chemotherapeutic agent may be selected from one or more of the group consisting of taxanes such as paclitaxel and docetaxel, platinum containing agents such as carboplatin, cisplatin and oxalipatin, gemcitabine, cyclophosphamide, abraxane, fluorouracil (5-FU), FolFox, Folfiri and Folfirinox.

In certain embodiments the secondary therapeutic compound is paclitaxel.

In certain embodiments, the TLR2 antagonists are for use in combination treatment with therapeutically effective amounts of two or more, for example two, secondary chemotherapeutic agents. For example, in one embodiment the TLR2 antagonist is for use in combination treatment with therapeutically effective amounts of two or more chemotherapeutic agents selected from the group consisting of paclitaxel, docetaxel, carboplatin, cisplatin, and gemcitabine. In one embodiment, the TLR2 antagonist is for use in combination treatment with therapeutically effective amounts of paclitaxel and carboplatin.

In certain embodiments the secondary therapeutic compound(s) is/are administered simultaneously, sequentially or separately to the TLR2 antibody or antigen binding fragment thereof. In certain embodiments the secondary therapeutic compound(s) is/are administered simultaneously to the TLR2 antibody or antigen binding fragment thereof.

### Administration

The TLR2 antagonist, for example TLR2 antibody or antigen binding fragment thereof, and, optionally, where appropriate other active agents such as chemotherapeutic agents, may be provided in the form of a pharmaceutical composition comprising a TLR2 antibody or antigen binding fragment thereof as described above and at least one pharmaceutically acceptable carrier, diluent, solubilizer, emulsifier, preservative and/or adjuvant. In certain embodiments, the composition includes a secondary therapeutic compound as described above, typically a chemotherapeutic agent, for example paclitaxel.

Examples of suitable pharmaceutical carriers include water, glycerol, ethanol and other GRAS reagents.

The TLR2 antagonist may be administered to a subject in need of treatment via any suitable route. As detailed herein, it is preferred that the composition is administered parenterally by injection or infusion. Examples of preferred routes for parenteral administration include, but are not limited to, intravenous, intracardial, intraarterial, intraperitoneal, intramuscular, intracavity, subcutaneous, transmucosal, inhalation and transdermal. Routes of administration may further include topical and enteral, for example, mucosal (including pulmonary), oral, nasal, rectal.

The composition may be delivered as an injectable composition. For intravenous, intramuscular, intradermal or subcutaneous application, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as sodium chloride injection, Ringer's injection or, Lactated Ringer's injection. Preservatives, stabilisers, buffers, antioxidants and/or other additives may be included, as required.

The composition may also be administered via microspheres, liposomes, other microparticulate delivery systems or sustained release formulations placed in certain tissues including blood.

The actual dose administered, and rate and time-course of administration, will depend on, and can be determined with due reference to, the nature and severity of the condition which is being treated, as well as factors such as the age, sex and weight of the subject to be treated and the route of administration. Further due consideration should be given to the properties of the composition, for example, its binding activity and invivo plasma life, the concentration of the antagonist in the formulation, as well as the route, site and rate of delivery.

Dosage regimens can include a single administration of the composition of the invention, or multiple administrative doses of the composition. The compositions can further be administered sequentially or separately with other therapeutics and medicaments which are used for the treatment of ovarian cancer.

The composition may be administered as a single dose or as repeated doses. Examples of dosage regimens which can be administered to a subject can be selected from the group comprising, but not limited to, 1µg/kg/day through to 20mg/kg/day, 1µg/kg/day through to 10mg/kg/day, 10µg/kg/day through to 1mg/kg/day.

A TLR2 antagonist may be orally administered to the subject, for example, at a dose of from about 1 mg/kg to about 10 mg/kg of the subject's body weight per day. In certain embodiments the dose of the TLR2 antagonist is from about 100 mg per day to about 1000 mg per day. In certain further embodiments the dose of the TLR2 antagonist is from about 200 mg per day to about 300 mg per day. In certain embodiments the TLR2 antagonist is administered to the subject parenterally with a dosage range of between about 0.001 mg/kg to 1.0 mg/kg of the subject's body weight. Typically, the TLR2 antagonist is administered to the subject for a time and under conditions sufficient to down regulate the level and/or activity of TLR2.

Typically, the subject is suffering from ovarian cancer. The subject may have one or more tumours. In certain embodiments the ovarian cancer is metastatic. In alternative embodiments the ovarian cancer is locally advanced. Alternatively, the ovarian cancer may be localised. In certain embodiments the subject may be at risk of developing ovarian cancer. Subjects at risk of developing ovarian cancer may be identified, for example, using genetic testing, in particular, by testing individuals having a family history of ovarian cancer. In certain embodiments, tumour cells from the subject are pre-screened, for example, at the earlier staging stage, for TLR2 expression. Treatment is administered to subjects with tumour cells showing TLR2 expression. This ensures the subject's tumour will respond to TLR2 antagonism. Typically, treatment is administered to subjects with later stage tumours.

### Candidate Compounds

As described herein, one aspect of the present invention provides an assay method for identifying compounds for use in the treatment or prevention of ovarian cancer. The method comprises identifying compounds which bind to and antagonise TLR2, for example, identifying compounds which bind the same epitope on TLR2 as that bound by the T2.5 and OPN-305 antibodies.

Typically, the candidate compound is an antibody or an antigen binding fragment thereof.

In a further aspect, the present invention extends to a screening method for the identification of compounds for use in treatment or prevention of ovarian cancer, the method comprising the steps of:
(a) providing candidate compounds, e.g. antibodies having binding specificity for TLR2 or antigen binding fragments thereof;
(b) contacting the candidate compounds with TLR2; and
(c) identifying compounds which antagonise TLR2;
wherein antagonism of TLR2 is indicative of utility of the compound in the treatment or prevention of ovarian cancer.

In a further aspect, the present invention extends to a screening method for the identification of compounds for use in treatment or prevention of ovarian cancer, the method comprising the steps of:
(a) providing candidate compounds which are antagonists of TLR2 function, e.g. antibodies having binding specificity for TLR2 or antigen binding fragments thereof;
(b) contacting the candidate compounds with TLR2; and
(c) identifying compounds which bind to TLR2 within the region of amino acid residues His318 (H, histidine), Pro320 (P, proline), Arg321 (R, arginine) or Gln321 (Q, glutamine), Tyr323 (Y, tyrosine), Lys347 (K, lysine), Phe349 (F, phenylalanine), Leu371 (L, leucine), Glu375 (E, glutamic acid), Tyr376 (Y, tyrosine), and His398 (H, histidine) of SEQ ID NO: 1 or SEQ ID NO:2;
wherein binding in this region is indicative of utility of the compound in the treatment or prevention of ovarian cancer.

In a further aspect, the present invention extends to a screening method for the identification of secondary therapeutic compounds for use with an antagonistic TLR2 antibody or antigen binding fragment thereof in treatment or prevention of ovarian cancer, the method comprising the steps of:
(a) screening candidate compounds, e.g. chemotherapeutic agents, more particularly chemotherapeutic agents suitable for treatment of ovarian cancer, for their ability to increase TLR2 expression when administered alone, i.e. without a TLR2 antagonist;
wherein an increase in TLR2 expression is indicative of utility of the compound as a secondary therapeutic compound for use with an antagonistic TLR2 antibody or antigen binding fragment thereof in treatment or prevention of ovarian cancer.

The precise format of the candidate compound screening assay may be varied by those skilled in the art using routine skill and knowledge. Combinatorial library technology provides an efficient way of testing a potentially vast number of different substances for the ability to bind to an epitope. The amount of candidate compound which may be added to an assay will normally be determined by trial and error depending upon the type of compound used. Typically, from about 0.01 to 100 nM concentrations of the candidate compound may be used, for example from 0.1 to 10 nM. Greater concentrations may be used when the candidate binding compound is a peptide. A candidate compound which has affinity and binding specificity for TLR2, e.g. the binding epitope described herein, may be isolated and/or purified and tested for its ability to antagonise TLR2 function. The compound may thereafter be manufactured and/or used to modulate TLR2 functional activity in the treatment or prevention of ovarian cancer.

### Sequence Identity

The present invention extends to the use of sequences having at least 80%, 85%, 90%, 95%, 97%, 98% or 99% amino acid sequence identity to the sequences of the TLR2 antagonists described herein. Such sequences or polypeptides may comprise a sequence which is substantially homologous to a polypeptide having the amino acid sequence of a TLR2 antagonist described herein, but may have a different amino acid sequence because of one or more deletions, insertions, or substitutions. The substantially homologous polypeptide may include 1, 2 or more amino acid alterations. Alternatively, or in addition, the substantially homologous polypeptide may consist of a truncated version of a TLR2 antagonist described herein which has been truncated by 1, 2 or more amino acids. In certain embodiments sequence identity is over a length of at least 20, 25, 30, 35 or 40 amino acids of the amino acid sequence in question. In certain embodiments sequence identity is over the complete length of the amino acid sequence in question (e.g. any one of SEQ ID NO:8, 9, 10, 11, 12 and/or 13).

A given amino acid may be replaced, for example, by a residue having similar physiochemical characteristics. Examples of such conservative substitutions include substitution of one aliphatic residue for another, such as Ile, Val, Leu, or Ala for one another; substitutions of one polar residue for another, such as between Lys and Arg, GIu and Asp, or GIn and Asn; or substitutions of one aromatic residue for another, such as Phe, Trp, or Tyr for one another. Other conservative substitutions, e.g., involving substitutions of entire regions having similar hydrophobicity characteristics, are well known.

Similarly, the nucleic acids for use herein may be variants that differ from a native DNA sequence because of one or more deletions, insertions or substitutions, but that encode a biologically active polypeptide.

As used herein, percentage amino acid sequence identity may be determined, using any method known to the person skilled in the art, for example, by comparing sequence information using the GAP computer program, version 6.0 described by Devereux et al. (Nucl. Acids Res. 12:387, 1984) and available from the University of Wisconsin Genetics Computer Group (UWGCG).

The present invention will now be described with reference to the following examples which are provided for the purpose of illustration and are not intended to be construed as being limiting on the present invention.

### Examples

### Example 1

### 1.1 Background

To identify novel treatment options the inventors developed an intra-peritoneal recurrent ovarian cancer animal model in athymic nude mice. Treatment with Paclitaxel results in a response but once treatment is terminated the tumours recur (Craveiro V et al. Cancer Med. 2013;2(6):751-62). The recurrent disease exhibits Paclitaxel resistance similar to that observed in patients (Craveiro V et al. Cancer Med. 2013;2(6):751-62; Alvero AB et al. Mol Cancer Ther. 2016;15(6):1279-90; Alvero AB et al.. Oncotarget. 2014;5(18):8703-15).

Using this model the inventors previously showed that compared to chemo-naive tumours, recurrent tumours that present post Paclitaxel treatment have a more mesenchymal and more stemness phenotype (Craveiro V et al. Cancer Med. 2013;2(6):751-62).

The objective of this example was to determine if the addition of OPN-305, a humanized anti-TLR2 antibody, to Paclitaxel could delay/prevent recurrence in this animal model. As detailed below OPN-305 was added to Paclitaxel either as part of primary treatment or given after the Paclitaxel regimen as part of maintenance treatment. The inventors also determined if this modality could inhibit the acquisition of a more mesenchymal and more stem-like phenotype.

### 1.2. Study materials and methods

### 1.2.1 Cell source, assurance, and culture

The generation of mCherry-labeled OCSC1-F2 ovarian cancer cells has been previously described (Craveiro V et al. Cancer Med. 2013;2(6):751-62; Alvero AB et al. Mol Cancer Ther. 2016;15(6):1279-90; Alvero AB et al.. Oncotarget. 2014;5(18):8703-15). Cells were maintained as low growing rate cultures and kept under 80% confluence. Cells were grown in RPMI supplemented with 10% FBS, 100 nM MEM non-essential amino acid solution, 2% sodium bicarbonate, 1 mM sodium pyruvate, 100 I.U./ml penicillin and 100 g/ml of streptomycin at 37°C in a 5% CO₂ atmosphere.

### 1.2.2 Test substance

TEST SUBSTANCE: OPN-305, batch # PD14590
FORMULATION: 10 mg/ml sterile solution in 25 mM sodium citrate, 125 mM sodium chloride, 6 mM citric acid, 0.01% (w/v) polysorbate 20, pH 5.5 at 10 mg/ml
DATE RECEIVED: Nov. 9, 2017
STORAGE CONDITIONS: 4oC
TEST SUBSTANCE: Paclitaxel (Hospira)
FORMULATION: for each ml: 6 mg Paclitaxel, 527 mg Polyoxyl 35 Castor oil, 49.7% (v/v) dehydrated alcohol, 2 mg citric acid
STORAGE CONDITIONS: room temperature

### 1.2.3 Determination of value of OPN-305 to prevent recurrence

The Yale University Institutional Animal Care and Use Committee approved all *in vivo* studies described (IACUC#2011-10063). During the study, the care and use of animals was in accordance with the principles outlined in the Guide for the Care and Use of Laboratory Animals, 9th Edition, 2011 (National Research Council).

Tumours were generated by injecting 4 × 10⁶ mCherry-labeled OCSC1-F2 cells (from culture conditions described above) into athymic nude mice. Cells were injected intraperitoneally (i.p.) in 200 µl of RPMI growth medium. Tumour growth was monitored q3-4d using the Invivo Imaging System FX PRO (Bruker Corp., Billerica, MA) via established methods (Craveiro V et al. Cancer Med. 2013;2(6):751-62; Alvero AB et al. Mol Cancer Ther. 2016;15(6):1279-90; Alvero AB et al Proceedings of the 104th Annual Meeting of the American Association for Cancer Research. 2013; Pizzonia J et al. Am J Reprod Immunol. 2012;67(1):84-90). Intra-peritoneal tumour burden was quantified by measuring total mCherry fluorescence area within the region of interest (ROI). mCherry fluorescence was captured with excitation at 440 nm and emission at 600 nm; images were adjusted to a min intensity to 75 and max intensity to 5000; intensity > 1.7X10³ was considered a positive signal. The fluorescence area from one or more lesions presenting with positive fluorescent signal were added to provide a total measure of tumour burden (ROI area) for each mouse.

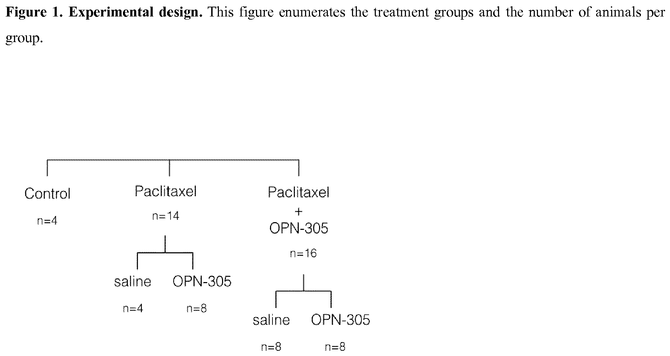

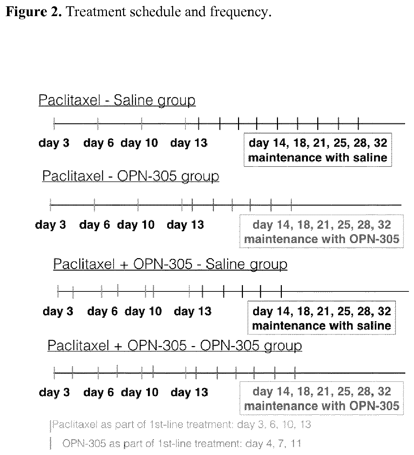

Treatment was initiated once tumours have ROI area > 2000. Paclitaxel was given i.p. at 12 mg/kg and OPN-305 was given i.p. at 10 mg/kg. The treatment groups, timing, and frequency is detailed in Figures 1 and 2.

Twenty-four hours after the last dose of maintenance treatment (day 33 of the study) all animals were evaluated and those with detectable tumours were euthanized and i.p. tumours were excised, weighed, and processed for further analysis (i.e. flow cytometry analysis for levels of myeloid-derived suppressor cells; flash-frozen for determination of cytokine/chemokine profile and western blot analysis for EMT and anti-apoptotic proteins; frozen in OCT for further IHC studies). Animals were euthanized with 2% isoflurane followed by cervical dislocation as per approved standard procedures.

Animals without detectable tumours on day 33 were not sacrificed and were continued to be monitored to determine time of recurrence for the calculation of progression-free interval. These animals were given one more dose of maintenance therapy and thus received a total of 7 doses of maintenance treatment. For complete responders (i.e. no detectable mCherry fluorescence by imaging at day 14, which is 24h after the last dose of Paclitaxel) progression-free interval is defined as the number of days for recurrent tumours to reach ROI area = 2,000; for partial responders, progression-free interval is defined as the number of days it took the measured ROI area at day 14 to double in size. Whereas animals without detectable tumours on day 33 were included in the analysis of tumour kinetics and progression-free interval, they were not included in the analysis of final i.p. tumour weight.

Prism (GraphPad Software, Version 6.0e) was used for statistical analysis. Statistical analysis used for each data set is specified in the results section. Differences were considered statistically significant if p<0.05.

### 1.2 Results

### 1.3.1 Effect on tumour kinetics

Of the 38 animals that were injected with cancer cells only 34 developed i.p. tumours and moved forward in the study. The 34 animals were randomized into three initial groups as part of PRIMARY TREATMENT: **Group 1:** Control no treatment (n=4); **Group 2:** Paclitaxel (n=14); and **Group 3:** Paclitaxel + OPN-305 (n=16) (Fig. 1). Paclitaxel was given i.p. at 12 mg/kg q3-4d (Mondays and Thursdays) for a total of 4 doses and in the group receiving OPN-305, the antibody was given i.p. at 10 mg/kg q3-4d (Tuesdays and Fridays) for a total of 3 doses (Fig. 2).

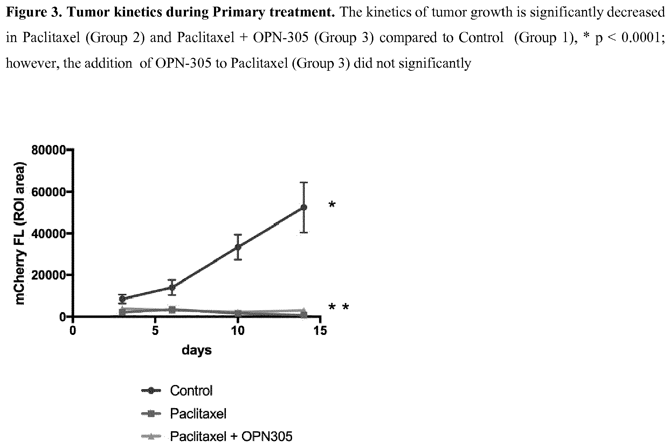

Overall, treatment with Paclitaxel (Group 2) significantly decreased tumour kinetics compared to no- treatment control (Group 1) but the addition of OPN-305 to Paclitaxel during primary treatment (Group 3) did not result in a significant difference compared to Paclitaxel alone (Group 2) (Fig. 3). Specifically, of the 14 animals that were treated with Paclitaxel alone as primary treatment, 1 exhibited progressive disease and thus was excluded from continuing to maintenance treatment. Similarly, of the 16 animals that were treated with Paclitaxel + OPN-305 as primary treatment 1 exhibited progressive disease and thus was excluded from continuing to maintenance treatment.

Thus there were a total of 28 animals that moved forward to maintenance treatment. After the 4^{th} dose of Paclitaxel, the 28 animals were re-randomized into the following groups as part of MAINTENANCE TREATMENT: **Group 1:** Paclitaxel-Saline (n=5); **Group 2:** Paclitaxel-OPN-305 (n=8); **Group 3:** Paclitaxel + OPN-305 - Saline (n=8); **Group 4:** Paclitaxel + OPN-305 - OPN-305 (n=7) (Fig.1). OPN-305 as maintenance treatment was given i.p. at 10 mg/kg q3-4d (Tuesdays and Fridays) for a total of 6 doses (Fig. 2). One animal in the Paclitaxel-Saline group was found dead in the middle of maintenance study, probably due to being accidentally caught in between the slots that hold the water bottle, and was subsequently excluded from the study.

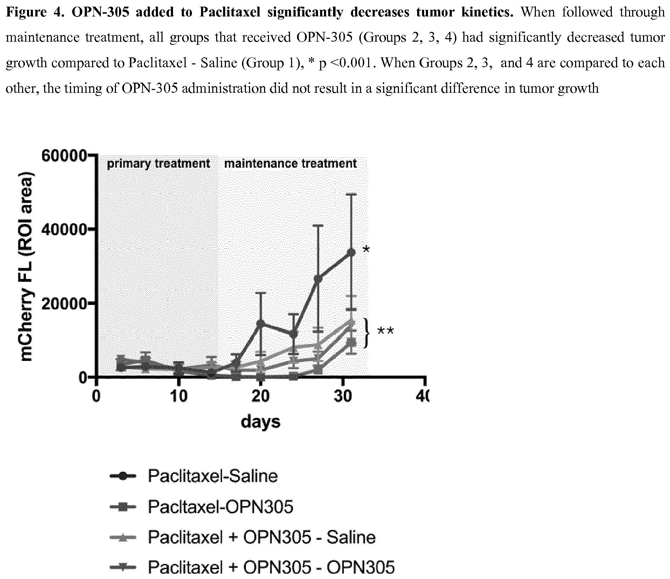

Analysis of ROI area showed that the addition of OPN-305, either as part of maintenance treatment (Group 2), or as part of the primary treatment (Group 3), or as part of both (Group 4) significantly decreased tumour kinetics compared to Paclitaxel alone (Group 1) (Fig. 4).

All 4 animals in the Paclitaxel-Saline group recurred and were sacrificed at the end of maintenance treatment (day 33). Six out of 8 animals in the Paclitaxel-OPN-305 group, 5 out of 8 animals in the Paclitaxel + OPN-305 - Saline group, and 5 out of 7 animals in the Paclitaxel + OPN-305 - OPN-305 group recurred and were sacrificed at the end of maintenance treatment.

### Example 2 Effect on tumour burden, and progression-free interval

In Example 1, the inventors performed their analysis comparing all 4 groups - (1) Paclitaxel-Saline; (2) Paclitaxel-OPN-305; (3) Paclitaxel + OPN-305 - Saline; and (4) Paclitaxel + OPN-305 - OPN-305.

Since an *in vivo* preliminary study (results not shown) implied the value of OPN-305 when given as maintenance treatment, the inventors performed the analysis combining data from the preliminary study and that of Example 1 in order to increase the sample sizes. The methods employed in the preliminary study were identical to those employed in Example 1.

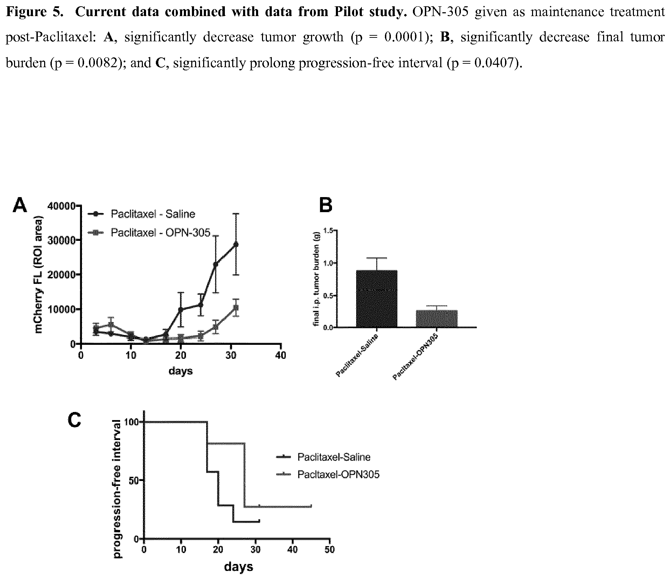

When combined, there were n =7 in the Paclitaxel-Saline group and n = 11 in the Paclitaxel - OPN-305 group. Using this data set the inventors observed that the administration of OPN-305 as maintenance treatment post Paclitaxel resulted in significantly decreased tumour kinetics, significantly reduced tumour burden, and significantly longer progression-free interval (Fig. 5A-C).

### Example 3 Effect on levels of intra-tumoural CD11b+/Gr1+ myeloid-derived suppressor cells

### Quantification of CD11b+/Gr1+ myeloid-derived suppressor cells

Collected tumours were dissociated using Collagenase and resulting single-cell suspension was analysed for expression of CD11b and Gr1 using Flow cytometry analysis. Cells were stained with anti-CDllb-FITC (eBioscience #11-0112-81) and anti-Gr1APC (eBioscience #17-5931). Matched isotype controls were used for each antibody to determine the gates. Data were acquired using BD FACSCalibur and analysed using Cellquest.

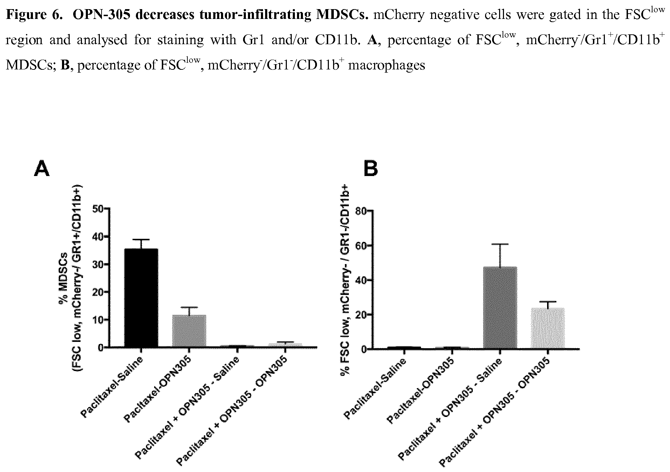

Analysis of intra-tumoural levels of CD11b+/Gr1+ MDSCs showed that compared to the tumours from the Paclitaxel-Saline treatment group, tumours from the Paclitaxel-OPN-305 treatment groups had less infiltrating MDSCs (Fig. 6A). Moreover, an even lower percentage of tumour-infiltrating MDSCs was observed in groups that received OPN-305 as part of primary treatment (i.e. Paclitaxel + OPN-305 - Saline and Paclitaxel + OPN-305 - OPN-305). In these two groups, there was a shift towards higher percentage of CD11b+/Gr1- macrophages (Fig. 6B).

### Example 4

### 4.1. Aim

This example shows data on the effect of OPN-305 on the molecular signature of ovarian cancer xenografts with focus on changes in the mesenchymal, anti-apoptotic, and stemness-associated proteins.

### 4.2. Study materials and methods

### 4.2.1 Western blot analysis

Total protein was extracted from tumour implants using detergent lysis method. Protein concentration was measured using the Bicinchoninic acid protein assay. Forty micrograms of protein was used in SDS-PAGE and immunoblotted for the proteins listed in the table below.

| **protein** | **antibody** | **dilution** |
|---|---|---|
| Twist1 | Santa Cruz Biotechnology #81416 | 1:200 |
| Slug | Cell Signaling Technology #9585 | 1:1,000 |
| Vimentin | Cell Signaling Technology #5741 | 1:1,000 |
| Klf4 | Cell Signaling Technology #4038 | 1:1,000 |
| Oct4 | Cell Signaling Technology #2750 | 1:1,000 |
| Bcl2a1 | Invitrogen #PA524561 | 1:1,000 |
| Bcl3 | Invitrogen #PA526346 | 1:1,000 |
| GAPDH | Sigma Aldrich #G8795 | 1:1,000 |

### 4.3. Study results

### 4.3.1 Effect on mesenchymal, anti-apoptotic, and sternness-associated proteins

The following samples were analysed: Paclitaxel-Saline group - animal #129, 138, and 141; Paclitaxel-OPN-305 group - animal #131, 134, 136, 137, 143; Paclitaxel+OPN-305 - Saline group - animal #144,147,148; Paclitaxel+OPN-305 - OPN-305 group - animal #153, 154, 155; control no treatment (NT) group - animal # 75.

Effect on mesenchymal proteins (Fig. 1A,B): Compared to the Paclitaxel-Saline group, 4 out of 5 animals in the Paclitaxel-OPN-305 group, 3 out of 3 animals in the Paclitaxel+OPN-305 - Saline group, and 2 out 3 animals in the Paclitaxel+OPN-305 - OPN-305 group showed lower levels of Twist1 expression. Slug was detected in only two samples, #141 and #154 and was undetectable in all the other samples tested. Vimentin expression was not notably different between the treatment groups.

Effect on anti-apoptotic proteins (Fig. 1C,D): Xenografts did not demonstrate notable signal from Bcl2a1 and Bcl3 immunoblots.

Effect on stemness-associated proteins (Fig. 1A,B): Klf4 and Oct4 expression was not markedly different between the treatment groups.

### Summary

The following can be concluded from this study:
(1) OPN-305 given as part of maintenance treatment post Paclitaxel significantly decreases tumour growth and tumour burden and prolongs progression-free interval.
(2) The addition of OPN-305 to Paclitaxel, irrespective of whether it is given as part of primary treatment or as part of maintenance treatment, significantly decreased tumour growth compared to Palitaxel alone.
(3) OPN-305 given as part of maintenance treatment post Paclitaxel significantly decreases the levels of tumour infiltrating MDSCs. However, earlier administration of OPN-305 (i.e. as part of primary treatment) resulted in a better inhibition of intra-tumoural MDSC influx.
(4) Treatment with OPN-305 was able to decrease the levels of Twist1 in ovarian tumours.

The inventors have thus demonstrated for the first time that maintenance therapy with a humanized anti-TLR2 antibody is able to modify the molecular and immune-phenotype of the tumour microenvironment and delay the progression of recurrent ovarian cancer. The inventors' data suggest that by inhibiting the TLR2 pathway, the inventors can block the capacity of the tumour microenvironment to recruit MDSC, which are critical in the process of tissue repair and are major inhibitors of anti-tumoural responses. These results suggest the value of TLR2 inhibitors for the treatment of ovarian cancer in general, and, in particular, as part of maintenance therapy in ovarian cancer.

## Claims

1. A composition comprising a Toll-like receptor 2 (TLR2) antagonist for use in the treatment of ovarian cancer in a subject.

2. The composition for use as claimed in claim 1, wherein said treatment is treatment or prevention of recurrent ovarian cancer.

3. The composition for use as claimed in claim 1, wherein said treatment is treatment or prevention of primary ovarian cancer.

4. The composition for use according to any one of the preceding claims, wherein said treatment is combination treatment with a chemotherapeutic agent and/or surgery, optionally wherein the TLR2 antagonist is for administration as maintenance therapy after cessation of treatment with a chemotherapeutic agent and/or after surgery.

5. The composition for use according to claim 4, wherein said combination treatment is combination treatment with one or more chemotherapeutic agents selected from taxanes and platinum containing chemotherapeutic agents, optionally wherein said combination treatment is combination treatment with paclitaxel.

6. The composition for use as claimed in any one of the preceding claims wherein said TLR2 inhibitor decreases Twist1 expression;
and/or
wherein said TLR2 inhibitor decreases levels of tumour infiltrating myeloid derived suppressor cells (MDSCs).

7. The composition for use according to any one of the preceding claims wherein the TLR2 antagonist is an antibody or an antigen binding fragment thereof; optionally wherein the antibody or antigen binding fragment thereof specifically binds to an epitope comprising leucine rich repeat regions 11 to 14 of TLR2.

8. The composition for use as claimed in claim 7 wherein the antibody or an antigen binding fragment thereof specifically binds to a non-continuous epitope comprising amino acid residues His318, Pro320, Arg321, Tyr323, Lys347, Phe349, Leu371, Glu375, Tyr376 and His398 of SEQ ID NO: 1, or comprising amino acid residues His318, Pro320, Gln321, Tyr323, Lys347, Phe349, Leu371, Glu375, Tyr376 and His398 of SEQ ID NO: 2.

9. The composition for use as claimed in claim 7 or claim 8 wherein the antibody or antigen binding fragment comprises a heavy chain variable region comprising a complementarity determining region (CDR) 1 region comprising the amino acid sequence of SEQ ID NO:3, a CDR2 region comprising the amino acid sequence of SEQ ID NO:4 and a CDR3 region comprising the amino acid sequence of SEQ ID NO:5, and/or a light chain variable region comprising a CDR1 region comprising the amino acid sequence of SEQ ID NO:6, a CDR2 region comprising the amino acid sequence Gly-Ala-Ser and a CDR3 region comprising the amino acid sequence of SEQ ID NO:7.

10. The composition for use as claimed in claim 9 wherein the antibody or antigen binding fragment comprises a light chain variable domain comprising an amino acid sequence of SEQ ID NO:10, or a sequence which has at least 90% amino acid sequence identity with SEQ ID NO:10, and/or a heavy chain variable domain comprising an amino acid sequence of SEQ ID NO:11, or a sequence which has at least 90% amino acid sequence identity with SEQ ID NO:1.

11. The composition for use as claimed in claim 9 or claim 10 wherein the antibody comprises a heavy chain comprising the amino acid sequence of SEQ ID NO:13, or a sequence which has at least 90% amino acid sequence identity to the amino acid sequence of SEQ ID NO:13, and/or a light chain comprising the amino acid sequence of SEQ ID NO:12, or a sequence which has at least 90% amino acid sequence identity to the amino acid sequence of SEQ ID NO:12, or an antigen binding fragment thereof.

12. The composition for use as claimed in claim 9 wherein the antibody or antigen binding fragment comprises a heavy chain variable region comprising the amino acid sequence of SEQ ID NO:8, or a sequence which has at least 90% amino acid sequence identity to the amino acid sequence of SEQ ID NO:8, and/or a light chain variable region comprising the amino acid sequence of SEQ ID NO:9, or a sequence which has at least 90% amino acid sequence identity to the amino acid sequence of SEQ ID NO:9.

13. The composition for use as claimed in any one of claims 7 to 12 wherein the antibody is a humanised version of anti-TLR2 antibody T2.5, or an antigen binding fragment thereof; optionally wherein the antibody is OPN-305, or an antigen binding fragment thereof.

14. A screening method for the identification of antibodies or antigen binding fragments thereof for use in treatment or prevention of ovarian cancer, the method comprising the steps of:
(a) providing candidate antibodies or antigen binding fragments thereof having binding specificity for TLR2;
(b) contacting the candidate antibodies or antigen binding fragments thereof with TLR2; and
(c) identifying antibodies or antigen binding fragments thereof which antagonise TLR2 function;
wherein antagonism of TLR2 function is indicative of utility of the antibody or antigen binding fragment thereof in the treatment or prevention of ovarian cancer.

15. The screening method as claimed in claim 14 wherein the method further includes step (d) and/or step (e):
wherein step (d) comprises:
(d) testing the ability of said antibodies or antigen binding fragments thereof to inhibit Twist1 expression in ovarian cancer cells; wherein ability to inhibit Twist1 expression in ovarian cancer cells is further indicative of utility of the antibodies or antigen binding fragments thereof in the treatment or prevention of ovarian cancer;
and step (e) comprises:
(e) testing the ability of said antibodies or antigen binding fragments thereof to inhibit infiltration of myeloid derived suppressor cells (MDSCs) into ovarian tumours; wherein ability to inhibit infiltration of myeloid derived suppressor cells (MDSCs) into ovarian tumours is further indicative of utility of the antibodies or antigen binding fragments thereof in the treatment or prevention of ovarian cancer.
